(19) **Europäisches Patentamt / European Patent Office / Office européen des brevets**

(11) **EP 4 553 848 A1**

(12) **EUROPEAN PATENT APPLICATION**
published in accordance with Art. 153(4) EPC

(43) Date of publication:
14.05.2025 Bulletin 2025/20

(51) International Patent Classification (IPC):
*G16H 50/20* (2018.01)

(21) Application number: 24754869.6

(22) Date of filing: 04.03.2024

(86) International application number:
PCT/CN2024/079795

(87) International publication number:
WO 2025/066032 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 CN 202311256171

(71) Applicants:
• Primedic (Jiangsu) Medical Science and
Technology Co., Ltd.
Zhenjiang, Jiangsu 212300 (CN)
• Metrax GmbH
78628 Rottweil (DE)

(72) Inventors:
• JING, Wei
Zhenjiang, Jiangsu 212300 (CN)
• HUANG, Hua
Zhenjiang, Jiangsu 212300 (CN)
• CHEN, Yilin
Zhenjiang, Jiangsu 212300 (CN)
• LIU, Sixing
Zhenjiang, Jiangsu 212300 (CN)
• WU, Qun
Zhenjiang, Jiangsu 212300 (CN)

(74) Representative: Huang, Liwei
Cäcilienstraße 12
40597 Düsseldorf (DE)

(54) **SMART AED-BASED FIRST-AID AUXILIARY METHOD, SYSTEM AND APPARATUS AND READABLE STORAGE MEDIUM**

(57) The present application discloses an intelligent AED first aid assistance method, system and apparatus, and a readable storage medium. The method includes: training and establishing a CPR mapping relationship according to a CPR action standard identifier with a feature of detecting and positioning a key point of implementer's human body posture and a feature of patient hemodynamic parameter, outputting a CPR mapping model, recognizing whether a first aid condition meets a standard before CPR implementation, and using the trained CPR mapping model to output CPR action standard feedback so as to perform instruction on CPR actions and to guide implementation of a CPR process. The system includes an intelligent AED apparatus, a first aid platform server, and a first aid medical terminal. The scheme combines deep learning, constructions of important models before and after the CPR implementation, and intelligent AED first aid assistance in which a full first aid process is accompanied by artificial intelligence-assisted guidance, so that the requirements of professionalism for an implementer in an emergency situation can be reduced, thus ensuring that CPR first aid steps can also be performed quickly and effectively on a patient in a sudden state and without professional staff.

Fig.1

**Description**

**CROSS-REFERENCE TO RELATED APPLICATIONS**

This application claims priority to Chinese patent application No. 202311256171.6 filed with CNIPA on September 27, 2023 and entitled "Intelligent AED First Aid Assistance Method, System and Apparatus, and Readable Storage Medium", the entire contents of which are incorporated herein by reference.

**TECHNICAL FIELD**

The present application relates to the technical field of cardio pulmonary resuscitation, in particular to an intelligent AED first aid assistance method, system and apparatus, and a readable storage medium, in which a full first aid process is accompanied by artificial intelligence-assisted guidance.

**BACKGROUND**

The statement in this section merely provides background information relevant to the present application and do not necessarily constitute the prior art.

Ventricular fibrillation occurs in 85%-90% of early cardiac arrest patients, and the most effective method of treating ventricular fibrillation is early defibrillation with an automated external defibrillator (AED). After the occurrence of ventricular fibrillation, it is most effective to defibrillate within 3-5 minutes. For every minute that defibrillation is delayed, a survival rate is decreased by 7%-10%. About 540,000 people die of cardiac arrests each year in China, and placing AED first-aid devices in public places is an important measure to reduce sudden deaths from cardiac arrests. AED external defibrillation is an important step in cardio pulmonary resuscitation (CPR). A person who uses an AED is an implementer of AED, CPR (hereinafter referred to as the implementer), and a person who receives AED external defibrillation is a recipient of AED, CPR (hereinafter referred to as a patient).

Currently, AEDs have been widely applied in the configuration of public places other than hospitals for timely rescue of patients with sudden cardiac arrests. A main structure of an AED is divided into electrical energy storage, a master control unit, a discharge unit, an electrode plate, etc. Since an AED device is a professional device that affects the safety of a patient's life, it usually requires an implementer who has undergone a high frequency of basic life support training and a professional medical worker to perform a first aid operation. However, after a sudden cardiac arrest occurs to a patient in a public place, there are not always professional first-aiders and medical workers to operate the AED, so there is a need for a method and an apparatus that can provide first aid assistance during the operation of the AED to perform corresponding first aid assistance to the implementer of the AED, CPR, and the needed first aid assistance should at least include the provision of guidance and instructions.

In the process of realizing the present application, the applicant found that the prior art in the scheme for providing guidance and instructions for AED use further suffers from at least the following problems.

1. In a current existing AED combined with CPR first aid process, only an AED containing a CPR sensor has corresponding CPR feedback, and an AED without a CPR sensor is far from enough for guidance and instructions in the CPR process.
2. Current existing CPR assistance technologies, especially an artificial intelligence technology using images, are mostly concerned only with guidance and instruction links for the CPR process, while guidance and environmental judgment links for CPR preparation and defibrillation stages combined with the AED use are still insufficient.
3. The assistance of current existing assistance methods for a CPR implementation stage mainly focuses on the use of an image recognition technology to recognize the implementer's pose, which is used for solving the standardization of CPR implementation action requirements, while ignoring a CPR receiving body, i.e., the patient's feedback under CPR, and improper or excessive CPR may lead to secondary injuries to the patient.

Therefore, how to solve the above problems of AED in the cardio pulmonary resuscitation process becomes a subject to be studied and solved in the present application

**SUMMARY**

The purpose of the present application is to provide an intelligent AED first aid assistance method, system and apparatus, and a readable storage medium.

In order to achieve the above purpose, a first aspect of the present application proposes an intelligent AED first aid assistance method, including the following steps:

S100, training and establishing a CPR mapping model, specifically, training and establishing a CPR mapping relationship according to a CPR action standard identifier with a feature of detecting and positioning a key point of implementer's human body posture and a feature of patient hemodynamic parameter, and outputting the CPR mapping model;

S200, recognizing whether a first aid condition meets a standard before CPR implementation, specifically, after an intelligent AED apparatus is enabled, performing a separation of a foreground and a background before the CPR implementation, recognizing and judging whether a current first aid environment meets the first aid standard with respect to the environmental background, and recognizing and judging whether a patient human body position feature meets the first aid standard with respect to the human body foreground; and

S300, providing instruction and guidance on CPR actions during the CPR implementation, specifically, during the CPR implementation, using a human body pose recognition model to locate a key point of an implementer and obtain the feature of detecting and positioning a key point of implementer's human body posture, establishing a human body skin reflection model, feeding back CPR feedback of a patient in real time on the feature of patient hemodynamic parameter, and combined with the feature of detecting and positioning a key point of implementer's human body posture and the feature of patient hemodynamic parameter, using the trained CPR mapping model to output CPR action standard feedback so as to perform instruction on CPR actions and to guide implementation of a CPR process.

A second aspect of the present application proposes an intelligent AED first aid assistance system used for intelligent AED first aid assistance in the method according to the first aspect. Its innovation points are that the system includes: an intelligent AED apparatus, a first aid platform server, and a first aid medical terminal;

the intelligent AED apparatus is an apparatus for external defibrillation with an AI recognition module, a CPR mapping model that is output after training and establishing a CPR mapping relationship according to a CPR action standard identifier by combining a feature of detecting and positioning the key point of implementer's human body posture and a feature of patient hemodynamic parameter is deployed in the AI recognition module, and the AI recognition module is configured to recognize whether a first aid condition meets a standard before CPR implementation, and provide instruction and guidance on CPR actions during the CPR implementation;

the first aid platform server communicates with the intelligent AED apparatus in a long-distance wireless mode, and the first aid platform server is used for receiving data uploaded by the intelligent AED apparatus, storing, forwarding and sending data required by a first aid doctor to the first aid medical terminal; and

the first aid medical terminal is used for displaying the received data and performing a real-time audio and video call with the intelligent AED apparatus.

A third aspect of the present application proposes an intelligent AED first aid assistance apparatus used for intelligent AED first aid assistance in the method according to the first aspect. The intelligent AED first aid assistance apparatus includes a master control module, an awakening detection module, a video collection module, an audio collection module, a power module, a communication module, an AI recognition module, a storage module, a voice broadcasting module, a video display module, and an early warning module;

the master control module is configured for information centralization, storage, analysis and decision making;

the awakening detection module is configured to automatically awaken an AED data collection when the intelligent AED apparatus is used;

the audio collection module is configured to collect real-time scene audio;

the video collection module is configured to collect a real-time scene video;

the power module is configured to supply power to other modules;

the AI recognition module is configured to recognize an electrode plate installation position and a first aid environment interference factor, perform a CPR quality analysis, recognize physiological data of a patient, and output error correction result information;

the storage module is configured to store AED running information, treatment information, detection information, and AI recognition feedback information;

the communication module is responsible for communicating with the first aid platform server and the first aid medical terminal;

the voice broadcasting module is configured to broadcast a voice prompt and audio of a video call;

the video display module is configured to display a video prompt and a video of a video call; and

the early warning module is configured to be responsible for warning when the first aid medical terminal does not respond to a first aid event.

A fourth aspect of the present application proposes a readable storage medium, storing a control program thereon. The control program, when executed by an AI recognition module, causes the AI recognition module to execute the steps of the intelligent AED first aid assistance method according to the first aspect.

Relevant contents of the present application are explained as follows.

1. Through the implementation of the above technical scheme of the present application, before the intelligent AED apparatus is deployed, the CPR mapping relationship is trained and established first according to the CPR action standard identifier with the feature of detecting and positioning a key point of implementer's human body posture and the feature of patient hemodynamic parameter, so as to provide the CPR mapping model for the subsequent CPR implementation. In the process of training and establishing the CPR mapping model, both CPR action norms of the implementer and the feedback of the patient under CPR are taken into account, so as to provide the correct and good instruction and guidance on CPR actions for the subsequent CPR implementation. This is a deep learning process, which may gradually improve accuracy and increase a recognition speed according to an increase in a training amount and the number of times the intelligent AED first aid assistance method is applied. The separation of the foreground and the background is performed before the CPR implementation, and recognition may be performed quickly. Whether the current first aid environment meets the first aid standard is recognized and judged with respect to the environmental background, whether the patient human body position feature meets the first aid standard is recognized and judged with respect to the human body foreground, and corresponding guidance and prompts are provided. Thus, it is ensured that the CPR environment is safe, and the patient pose can meet the first aid requirements. Necessary technical recognition and guidance are provided for the implementation of CPR, so as to avoid secondary injuries to the patient, and to avoid an effect reduction or ineffectiveness of CPR. During CPR implementation, the human body pose recognition model is then used to locate the key point of the implementer and obtain the feature of detecting and positioning a key point of implementer's human body posture, which provides corresponding feature parameters for whether a rescuer's operation position, CPR press position, CPR press pose, CPR press depth, CPR press frequency, the number of CPR presses, etc. are standard. The human body skin reflection model is established, the CPR feedback of the patient is fed back in real time on the feature of patient hemodynamic parameter, which provides corresponding feature parameters for a very important human body cardiac activity and hemodynamic activity state of the patient during CPR implementation, and the feature of detecting and positioning a key point of implementer's human body posture and the feature of patient hemodynamic parameter are combined. Real-time feedback may also allow a third party other than the implementer to intervene to better provide additional instruction and cooperation according to the human body cardiac activity and hemodynamic activity state of the patient. Then the trained CPR mapping model is used to output the CPR action standard feedback so as to perform instruction on CPR actions and to guide implementation of the CPR process. The implementation of the above scheme combines deep learning, constructions of important models before and after the CPR implementation, and intelligent AED first aid assistance in which a full first aid process is accompanied by artificial intelligence-assisted guidance, so that the requirements of professionalism for the implementer in an emergency situation can be reduced. The implementer who has undergone a low frequency of basic life support training can also quickly perform CPR first aid on the patient, thus ensuring that the CPR first aid can be performed quickly and effectively on the patient in a sudden state and without professional staff to increase a patient survival rate and improve a first aid effect. Accurate and complete treatment information is also provided for subsequent treatment, so as to further ensure the life safety and health protection of the patient.

Due to the utilization of the above scheme, the present application has the following advantages and effects compared to the prior art.

1. Through the implementation of the technical scheme of the present application, before the intelligent AED apparatus is deployed, the CPR mapping relationship is trained and established first according to the CPR action standard identifier with the feature of detecting and positioning a key point of implementer's human body posture and the feature of patient hemodynamic parameter, so as to provide the CPR mapping model for the subsequent CPR implementation. In the process of training and establishing the CPR mapping model, both CPR action norms of the implementer and the feedback of the patient under CPR are taken into account, so as to provide the correct and good instruction and guidance on CPR actions for the subsequent CPR implementation. This is a deep learning process, which may gradually improve accuracy and increase a recognition speed according to an increase in a training amount and the number of times the intelligent AED first aid assistance method is applied.

2. Through the implementation of the technical scheme of the present application, the separation of the foreground and the background is performed before the CPR implementation, and recognition may be performed quickly. Whether the current first aid environment meets the first aid standard is recognized and judged with respect to the environmental background, whether the patient human body position feature meets the first aid standard is recognized and judged with respect to the human body foreground, and corresponding guidance and prompts are provided. Thus, it is ensured that the CPR environment is safe, and the patient pose can meet the first aid requirements. Necessary technical recognition and guidance are provided for the implementation of CPR, so as to avoid secondary injuries to the patient, and to avoid an effect reduction or ineffectiveness of CPR.

3. Through the implementation of the technical scheme of the present application, during CPR implementation, the human body pose recognition model is then used to locate the key point of the implementer and obtain the feature of detecting and positioning a key point of implementer's human body posture, which provides corresponding feature parameters for whether a rescuer's operation position, CPR press position, CPR press pose, CPR press depth, CPR press frequency, the number of CPR presses, etc. are standard. The human body skin reflection model is established, the CPR feedback of the patient is fed back in real time on the feature of patient hemodynamic parameter, which provides corresponding feature parameters for a very important human body cardiac activity and hemodynamic activity state of the patient during CPR implementation, and the feature of detecting and positioning a key point of implementer's human body posture and the feature of patient hemodynamic parameter are combined. Real-time feedback may also allow a third party other than the implementer to intervene to better provide additional instruction and cooperation according to the human body cardiac activity and hemodynamic activity state of the patient. Then the trained CPR mapping model is used to output the CPR action standard feedback so as to perform instruction on CPR actions and to guide implementation of the CPR process.

4. To sum up, the implementation of the above scheme combines deep learning, constructions of important models before and after the CPR implementation, and intelligent AED first aid assistance in which a full first aid process is accompanied by artificial intelligence-assisted guidance, so that the requirements of professionalism for the implementer in an emergency situation can be reduced. The implementer who has undergone a low frequency of basic life support training can also quickly perform CPR first aid on the patient, thus ensuring that the CPR first aid can be performed quickly and effectively on the patient in a sudden state and without professional staff, as well as incorporating real-time body feedback from the patient throughout the entire process to improve a patient survival rate and a first aid effect. Accurate and complete treatment information is also provided for subsequent treatment, so as to further ensure the life safety and health protection of the patient.

## BRIEF DESCRIPTION OF THE DRAWINGS

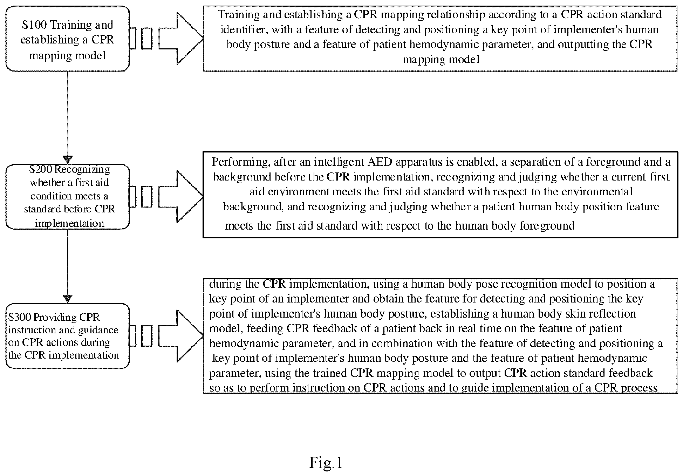

Fig. 1 is a schematic flow diagram of an intelligent AED first aid assistance method of an embodiment of the present application.

Fig. 2 is a schematic detailed flow diagram of an intelligent AED first aid assistance method of an embodiment of the present application.

Fig. 3 is a schematic diagram of an intelligent AED first aid assistance system of an embodiment of the present application.

Fig. 4 is a schematic diagram of an intelligent AED first aid assistance apparatus of an embodiment of the present application.

Fig. 5 is a flow diagram of work of a system of an embodiment of the present application.

Fig. 6 is a schematic diagram of a color camera collecting an rPPG signal of a human body.

Fig. 7 is a schematic diagram of a dichromatic reflection model.

Fig. 8 is an oscillogram of ECG and PPG.

## DETAILED DESCRIPTION OF THE EMBODIMENTS

In order to make above objects, characteristics and advantages of the present application more obvious and understandable, the detailed description of the present application is described in detail below in combination with accompanying drawings. Many specific details are set forth in the following description to facilitate full understanding of the present application. However, the present application can be implemented in many other ways different from those described here, and those skilled in the art can make similar improvements without violating the connotation of the present application. Therefore, the present application is not limited by specific embodiments disclosed below.

Terms used herein are intended only to describe particular embodiments and are not intended to be limitations of the present case. Singular forms such as "one", "this", "such", "present" and "the", as used herein, also contain plural forms.

Terms "first", "second", etc., as used herein, are not intended to refer specifically to order or precedence, nor are they intended to limit the present case, but are intended only to distinguish components or operations described in the same technical terms.

As used herein, "connect" or "locate" may refer to two or more components or apparatuses being in direct physical contact with each other or in indirect physical contact with each other, or to two or more components or apparatuses being operated or acted upon by each other.

"Contain", "include", "have", etc., as used herein, are all open-ended terms, i.e., they are meant to include, but not limited to.

Unless otherwise indicated, terms used herein have ordinary meanings given to each term as it is used in this field, in the content of the present case, and in the special content. Certain terms used to describe the present case will be discussed below or elsewhere in this specification to provide additional guidance to those skilled in the art with respect to the description of the present case.

The present application aims to provide intelligent AED first aid assistance in which a full first aid process is accompanied by artificial intelligence-assisted guidance, which combines deep learning, and constructions of important models before and after CPR implementation, can reduce the requirements of professionalism for an implementer in an emergency situation, and enables the implementer who has undergone a low frequency of basic life support training to also quickly perform CPR first aid on a patient, thus ensuring that the CPR first aid can be performed quickly and effectively on the patient in a sudden state and without professional staff, as well as incorporating real-time body feedback from the patient throughout the entire process to improve a patient survival rate and a first aid effect.

Embodiment 1, Embodiment 1 of the present application proposes an intelligent AED first aid assistance method, and the intelligent AED first aid assistance method includes the following steps.

S100, a CPR mapping model is trained and established, specifically, a CPR mapping relationship is trained and established according to a CPR action standard identifier with a feature of detecting and positioning a key point of implementer's human body posture and a feature of patient hemodynamic parameter, and the CPR mapping model is output.

S200, whether a first aid condition meets a standard is recognized before CPR implementation, specifically, after an intelligent AED apparatus is enabled, a separation of a foreground and a background is performed before the CPR implementation, whether a current first aid environment conform to the first aid standard is recognized and judged with respect to the environmental background, and whether a patient human body position feature meets the first aid standard is recognized and judged with respect to the human body foreground.

S300, instruction and guidance on CPR actions are provided during the CPR implementation, specifically, during the CPR implementation, a human body pose recognition model is used to locate a key point of an implementer and obtain the feature of detecting and positioning a key point of implementer's human body posture, a human body skin reflection model is established, CPR feedback of a patient is fed back in real time on the feature of patient hemodynamic parameter, and combined with the feature of detecting and positioning a key point of implementer's human body posture and the feature of patient hemodynamic parameter, the trained CPR mapping model is used to output CPR action standard feedback so as to perform instruction on CPR actions and to guide implementation of a CPR process.

In the above step S100 of training and establishing the CPR mapping model, before deploying the intelligent AED apparatus, the CPR mapping relationship is first trained and established according to the CPR action standard identifier with the feature of detecting and positioning a key point of implementer's human body posture and the feature of patient hemodynamic parameter, so as to provide the CPR mapping model for the subsequent CPR implementation. In the process of training and establishing the CPR mapping model, both CPR action norms of the implementer and the feedback of the patient under CPR are taken into account, so as to provide the correct and good instruction and guidance on CPR actions for the subsequent CPR implementation. This is a deep learning process, which may gradually improve accuracy and increase a recognition speed according to an increase in a training amount and the number of times the intelligent AED first aid assistance method is applied.

In above step S200 of recognizing whether the first aid condition meets the standard before the CPR implementation, the separation of the foreground and the background is performed before the CPR implementation, and recognition may be performed quickly. Whether the current first aid environment meets the first aid standard is recognized and judged with respect to the environmental background, whether the patient human body position feature meets the first aid standard is recognized and judged with respect to the human body foreground, and corresponding guidance and prompts are provided. Thus, it is ensured that the CPR environment is safe, and the patient pose can meet the first aid requirements. Necessary technical recognition and guidance are provided for the implementation of CPR, so as to avoid secondary injuries to the patient, and to avoid an effect reduction or ineffectiveness of CPR.

In above step S300 of providing the instruction and guidance on CPR actions during the CPR implementation, during the CPR implementation, the human body pose recognition model is then used to locate the key point of the implementer and obtain the feature of detecting and positioning a key point of implementer's human body posture, which provides corresponding feature parameters for whether a rescuer's operation position, CPR press position, CPR press pose, CPR press depth, CPR press frequency, the number of CPR presses, etc. are standard. The human body skin reflection model is established, the CPR feedback of the patient is fed back in real time on the feature of patient hemodynamic parameter, which provides corresponding feature parameters for a very important human body cardiac activity and hemodynamic activity state of the patient during CPR implementation, and the feature of detecting and positioning a key point of implementer's human body posture and the feature of patient hemodynamic parameter are combined. Real-time feedback may also allow a third party other than the implementer to intervene to better provide additional instruction and cooperation according to the human body cardiac activity and hemodynamic activity state of the patient. Then the trained CPR mapping model is used to output the CPR action standard feedback so as to perform instruction on CPR actions and to guide implementation of the CPR process.

In Embodiment 1 of the present application, the first aid assistance method further includes starting a remote AI first aid function after the intelligent AED apparatus is enabled, connecting the intelligent AED apparatus to a remote doctor through a first aid platform server, and uploading foreground data and background data before the CPR implementation as well as implementer data and patient data during the CPR implementation to the first aid platform server, so that the remote doctor participates in CPR first aid with audio and a video in a whole process before and during the CPR implementation. The remote doctor remotely instructs the implementer at the scene to execute effective CPR, the correct use of the AED, and other operations, so as to make up for the urgent emergency scene where the implementer is unable to take care of all the circumstances. In addition, as a professional remote doctor who participates in the whole process of CPR first aid, he or she may provide references and relevant opinions for the subsequent treatment of the patient after CPR first aid to further ensure that the health of the patient can be well treated.

Specifically, an AED data collection is automatically awakened after the intelligent AED apparatus is enabled, an AI identification of the foreground data, the background data, the implementer data, and the patient data is performed, an identification output is performed according to AI identification results corresponding to the CPR mapping model, and voice guidance and a screen display are provided in performing the instruction on CPR actions. Therefore, the remote doctor in a remote area can synchronously see a series of first aid data about the first aid environment, first aid conditions, implementer's action, patient's feedback, etc. on the first aid scene, which is more intuitive and fast, and allows the remote doctor to understand the relevant first aid process at the first time so that the remote doctor can participate in the CPR first aid more comprehensively way. At the same time, these identifiers and outputs also enable the implementer at the first aid scene to obtain more comprehensive, more accurate and faster CPR guidance as a way to provide more professional AED first aid to the patient.

Embodiment 1 of the present application is further described below in more detail with one of the preferred embodiments.

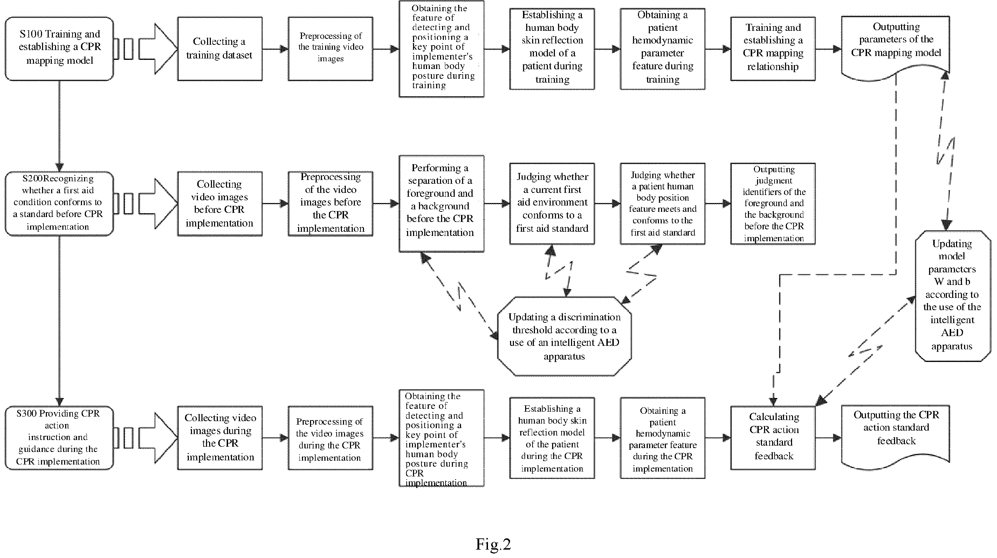

The intelligent AED first aid assistance method of this preferred embodiment contains each of the following detailed steps as shown in Fig. 2.

S100, a CPR mapping model is trained and established, specifically, a CPR mapping relationship is trained and established according to a CPR action standard identifier with a feature of detecting and positioning a key point of implementer's human body posture and a feature of patient hemodynamic parameter, and the CPR mapping model is output.

Step S100 of training and establishing the CPR mapping model specifically includes the following steps.

S110, a training data set is collected, video images of the implementer and the patient during training are collected, and the CPR action standard identifier is gathered.

S120, the training video images are preprocessed, and specifically, greying and filtering preprocessing is performed on the collected video image.

S130, the feature of detecting and positioning a key point of implementer's human body posture during training is obtained, specifically, implementer human body pose key point detection and localization are performed, and the feature of detecting and positioning a key point of implementer's human body posture is obtained.

S140, the human body skin reflection model of the patient during training is established, and specifically, an optical rPPG technology is used to establish the human body skin reflection model during training.

S150, the feature of patient hemodynamic parameter during training is obtained, and specifically, the human body skin reflection model is used to further calculate and obtain the feature of patient hemodynamic parameter during training.

S160, the CPR mapping relationship is trained and established, and specifically, the CPR mapping relationship is trained and established according to the CPR action standard identifier with the feature of detecting and positioning a key point of implementer's human body posture and the feature of patient hemodynamic parameter during training.

S170, a parameter of the CPR mapping model is output, and specifically, the parameter of the CPR mapping model is output according to the CPR mapping relationship.

Through the implementation of the process of training and establishing the CPR mapping model above, the problem of ignoring the feedback of the patient under CPR in the process of normalizing the requirements of CPR implementation actions is solved, which makes it possible to combine the feature of detecting and positioning a key point of implementer's human body posture and the feature of patient hemodynamic parameter in the output parameter of the CPR mapping model. In addition, an optical rPPG technology is used to establish the human body skin reflection model during training, so that a training result is more in line with various situations that will occur to the implementer and the patient during the actual CPR first aid implementation, and then the final CPR mapping model is more reasonable and accurate, which provides a better intelligent AED first aid assistance during the CPR first aid implementation.

S200, whether a first aid condition meets a standard is recognized before CPR implementation, specifically, after an intelligent AED apparatus is enabled, a separation of a foreground and a background is performed before the CPR implementation, whether a current first aid environment meets the first aid standard is recognized and judged with respect to the environmental background, and whether a patient human body position feature meets the first aid standard is recognized and judged with respect to the human body foreground.

Step S200 of recognizing whether the first aid condition meets the standard before the CPR implementation specifically includes the following steps.

S210, video images before the CPR implementation are collected, specifically, the video image of an environment in which the AED device is located and the video image of a patient human body before the CPR implementation are collected.

S220, the video images before the CPR implementation are preprocessed, specifically, greying and filtering preprocessing is performed on the collected video image of the environment in which the AED device is located and the video image of the patient human body.

S230, the separation of the foreground and the background before the CPR implementation is performed, and specifically, the environmental background is separated from the foreground human body. In step S230 of performing the separation of the foreground and the background before the CPR implementation, the separation of the foreground and the background is performed by using a deep low-rank model; the background is extracted with a residual component, and background residual changes during defibrillation are compared; and the patient human body position feature is automatically traced with a low-rank component.

S240, whether the current first aid environment meets the first aid standard is judged, specifically, an environmental interference feedback identifier before the CPR implementation is calculated according to the environmental background, and whether the environmental background meets the first aid standard is judged. In step S240 of judging whether the current first aid environment meets the first aid standard, whether an output photography distance is suitable and whether the environment is safe and dry are judged according to the environmental interference feedback identifier.

S250, whether the patient human body position feature meets the first aid standard is judged, specifically, the patient human body position feature of the foreground human body is extracted, and whether the patient meets the first aid standard is judged according to the patient human body position feature. In step S250 of judging whether the patient human body position feature meets the first aid standard, whether a patient pose meets a requirement, whether clothing meets a requirement, and whether an electrode plate placing position is correct are judged according to the patient human body position feature.

S260, judgment identifiers of the foreground and the background before the CPR implementation are output.

Through the implementation of the above process before the CPR implementation, whether the first aid condition meets the standard may be intelligently judged for the implementer before the CPR is performed, so as to ensure the safety of the first aid environment for the implementer, avoid the patient from being injured twice, and provide the correct intelligent assistance for the implementer who implements the CPR in an emergency situation as a way to reduce the burden of the implementer. At the same time, the above process before the CPR implementation may more quickly and accurately separate the foreground and background and judge and recognize them respectively with a high accuracy rate, which may exclude many interfering factors so as to further secure the safety of the first aid environment.

Through the implementation of the above process before the CPR implementation, the precision, accuracy and efficiency of the separation of foreground and background are further enhanced, and a more rational and rapid means is adopted to recognize whether the first aid condition meets the standard before the CPR implementation.

S300, instruction and guidance on CPR actions are provided during the CPR implementation, specifically, during the CPR implementation, a human body pose recognition model is used to locate a key point of an implementer and obtain the feature of detecting and positioning a key point of implementer's human body posture, a human body skin reflection model is established. CPR feedback of a patient is fed back in real time on the feature of patient hemodynamic parameter, and combined with the feature of detecting and positioning a key point of implementer's human body posture and the feature of patient hemodynamic parameter, the trained CPR mapping model is used to output CPR action standard feedback so as to perform instruction on CPR actions and to guide implementation of a CPR process.

Step S300 of providing the instruction and guidance on CPR actions during the CPR implementation specifically includes the following steps.

S310, the video images during the CPR implementation are collected, and specifically, the video image of the implementer and the video image of the patient are collected during the CPR implementation.

S320, the video images during the CPR implementation are preprocessed, and specifically, greying and filtering preprocessing is performed on the collected video image of the implementer and the video image of the patient.

S330, the feature of detecting and positioning a key point of implementer's human body posture during the CPR implementation is obtained, specifically, implementer human body pose key point detection and localization are performed, and the feature of detecting and positioning a key point of implementer's human body posture during the CPR implementation is obtained.

S340, the human body skin reflection model of the patient during the CPR implementation is established, and specifically, the optical rPPG technology is used to establish the human body skin reflection model during the CPR implementation.

S350, the feature of patient hemodynamic parameter during the CPR implementation is obtained, and specifically, the human body skin reflection model is used to further calculate and obtain the feature of patient hemodynamic parameter during the CPR implementation.

S360, the CPR action standard feedback is calculated, and specifically, the trained CPR mapping model is used to calculate the CPR action standard feedback with the feature of detecting and positioning a key point of implementer's human body posture and the feature of patient hemodynamic parameter during the CPR implementation.

S370, the CPR action standard feedback is output, a CPR action standard is fed back to the implementer who is implementing CPR, the instruction on CPR actions is performed, and the implementation of the CPR process is guided.

Through the above specific implementation of providing the instruction and guidance on CPR actions during the CPR implementation, the action of the implementer and the response of the patient during the actual CPR implementation may be better obtained, and then combined with the CPR mapping model, more reasonable and accurate feedback is obtained, so as to perform the correct and rapid instruction on CPR actions to reduce the professional requirements for the implementer, and to ensure that the patient can be correctly rescued.

For the establishment of the human body skin reflection model in steps S100 and S300, based on a remote photovoltaic volumetric pulse wave, a subtle color change caused by a pulse on a surface of a human body skin is detected by using a multi-wavelength RGB camera in a webcam to realize non-contact monitoring of a human body cardiac activity and a hemodynamic parameter so as to establish the human body skin reflection model. A principle of using the rPPG technology to build the skin reflection optical model is that: a spectrum and color channel sensitivity of a light source formed by diffuse reflection of the skin are received by the camera, and the color is skin deformation/specular reflection change caused by the operation of the RPC implementer and a subtle color change caused by a pulse. The color change, i.e., the skin diffuse reflection light spectrum, is absorbed and reflected by the epidermal skin tissue and internal bones to varying degrees, which may reflect the hemodynamic activity state of the human body, and the analysis of the received light by using an artificial intelligence method may obtain feedback on the human body hemodynamic parameter.

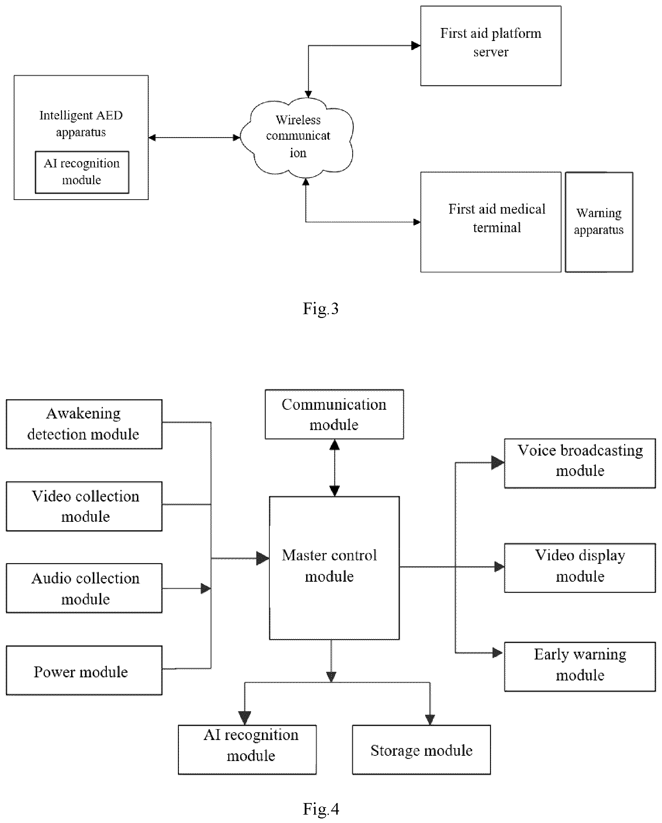

Embodiment 2, as shown in Fig. 3, Embodiment 2 of the present application proposes an intelligent AED first aid assistance system, which includes: an intelligent AED apparatus, a first aid platform server, and a first aid medical terminal.

The intelligent AED apparatus is an apparatus for external defibrillation with an AI recognition module. A CPR mapping model that is output after training and establishing a CPR mapping relationship according to a CPR action standard identifier by combining a feature of detecting and positioning the key point of implementer's human body posture and a feature of patient hemodynamic parameter is deployed in the AI recognition module, and the AI recognition module is configured to recognize whether a first aid condition meets a standard before CPR implementation, and provide instruction and guidance on CPR actions during the CPR implementation.

The first aid platform server communicates with the intelligent AED apparatus in a long-distance wireless mode, and the first aid platform server is used for receiving data uploaded by the intelligent AED apparatus, storing, forwarding and sending data required by a first aid doctor to the first aid medical terminal.

The first aid medical terminal is used for displaying the received data and performing a real-time audio and video call with the intelligent AED apparatus.

In Embodiment 2 of the present application, the long-distance wireless mode includes, but is not limited to, a cellular network, Wi-Fi, and other modes. The first aid medical terminal may be a mobile terminal, such as a smartphone, a tablet, etc., with real-time networked communication and video call functions. Or it may be a laptop computer, and a PC with a webcam-microphone installed, which is used for remotely instructing on-scene personnel to perform effective CPR, the use of an AED, and other operations. A warning apparatus is configured at the first aid medical terminal and responsible for warning when the first aid medical terminal does not respond to a first aid event.

Embodiment 3, as shown in Fig. 4, Embodiment 3 of the present application proposes an intelligent AED first aid assistance apparatus used for intelligent AED first aid assistance in the method according to Embodiment 1 of the present application. The intelligent AED first aid assistance apparatus includes a master control module, an awakening detection module, a video collection module, an audio collection module, a power module, a communication module, an AI recognition module, a storage module, a voice broadcasting module, a video display module, and an early warning module.

The master control module is configured for information centralization, storage, analysis and decision making.

The awakening detection module is configured to automatically awaken an AED data collection when the intelligent AED apparatus is used.

The audio collection module is configured to collect real-time scene audio.

The video collection module is configured to collect a real-time scene video.

The power module is configured to supply power to other modules.

The AI recognition module is configured to recognize an electrode plate installation position and a first aid environment interference factor, perform a CPR quality analysis, recognize physiological data of a patient, and output error correction result information.

The storage module is configured to store AED running information, treatment information, detection information, and AI recognition feedback information.

The communication module is responsible for communicating with the first aid platform server.

The voice broadcasting module is configured to broadcast a voice prompt and audio of a video call.

The video display module is configured to display a video prompt and a video of a video call.

The early warning module is configured to be responsible for warning when the first aid medical terminal does not respond to a first aid event.

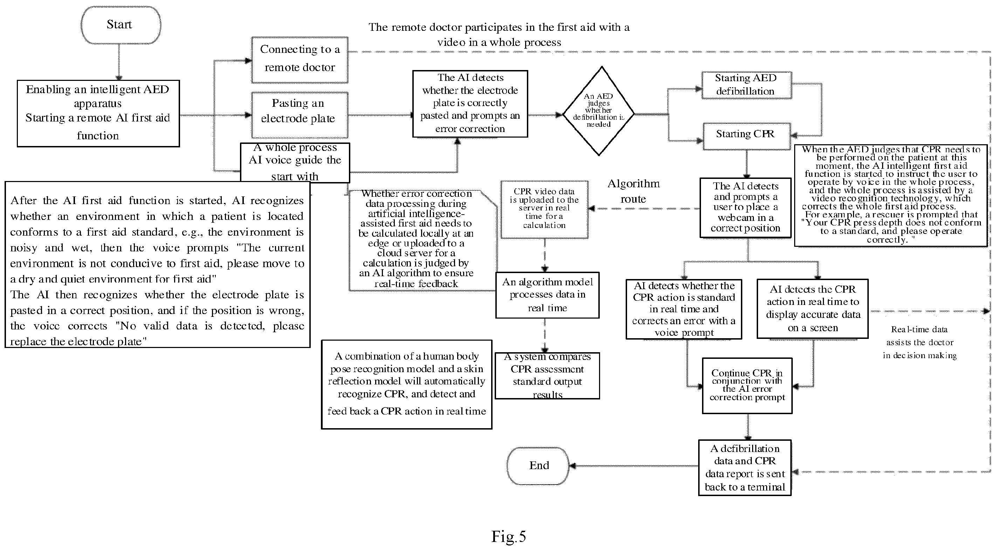

The practical application of the intelligent AED first aid assistance system of the present application is described below in conjunction with a system workflow shown in Fig. 5.

The system workflow of the intelligent AED first aid assistance system is as follows.

(1) AED data collection is automatically awakened after an intelligent AED first aid assistance apparatus is enabled, and a remote AI first aid function is started.

(2) After the intelligent first aid assistance function is enabled, the AI recognition module of the intelligent AED first aid assistance apparatus is automatically enabled to obtain multi-angle real-time images of the first aid scene through a 360-degree movable webcam so as to collect relevant data for the AI image recognition.

(3) After the intelligent AED first aid assistance apparatus is enabled, an emergency event is transmitted to the first aid medical terminal through the first aid platform server, a doctor is notified through a text message, a system message prompt, and other modes, and the warning apparatus provides double insurances for the doctor to respond to the emergency event quickly.

(4) The AI recognition module judges whether the patient currently needs a CPR operation, and if not, AED defibrillation is directly performed.

(5) When the CPR operation needs to be performed on the patient, the AI recognition module first recognizes whether a current first aid environment meets a standard.

(6) After confirming that the current first aid environment meets the first aid standard, the AI recognition module recognizes whether an electrode plate of the patient is correctly placed.

(7) During the CPR process, the AI recognition module will combine a plurality of algorithm models to recognize whether the CPR operation of the implementer meets the standard.

(8) The voice broadcasting module and the video display module will provide voice and video prompts for error corrections fed back after data processing by the AI algorithm models.

(9) During the first aid process, the intelligent AED first aid assistance apparatus establishes a video call with the first aid medical terminal through the first aid platform server.

(10) The remote doctor may see a result of AI data processing and analysis in real time on a screen at the first aid medical terminal and make a right decision.

(11) The storage module stores AED running information, treatment information, detection information, and AI recognition feedback information in real time.

(12) The communication module uploads collected AED running data, monitoring data, real-time scene audio and video data, data fed back by the AI recognition module, and localization data in real time to the first aid platform server and forwards them to the first aid medical terminal.

(13) The first aid medical terminal displays in real time audio and video information, localization information, and AED data sent by the AED intelligent module and information fed back by the AI recognition module, and sends audio and video data to the AED through the first aid platform server.

(14) The intelligent module performs a data communication with the first aid platform server through the communication module, obtains the audio and video data sent by the first aid platform server, and performs broadcasting through the voice broadcasting module and displaying through the video display module.

The AI recognition module in the intelligent AED apparatus is described in further detail below.

The CPR process in conjunction with the AED focuses on recognizing whether the current first aid environment meets the standard and recognizing whether the electrode plate of the patient is correctly placed corresponding to procedures (5) and (6), and recognizing whether the CPR operation meets the standard corresponding to procedure (7).

The AI recognition module has main functions as follows.

1. For the stage before the CPR implementation, the separation of the foreground and the background is performed by using a deep low-rank model, a human body outline of the foreground patient is extracted separately, and the patient human body position feature is automatically traced with a low-rank component. According to these features, whether a patient pose meets the requirements, whether clothing meets the requirements, and whether an electrode plate placement position is correct are output. The background is extracted with a residual component, and background residual changes during defibrillation are compared. An environmental interference feedback identifier is calculated, and whether an output photography distance is suitable and whether the environment is safe and dry are judged.

2. For the CPR implementation stage, the human pose key point model is used to localize the key point of the implementer, the optical rPPG technology is used to establish the human body skin reflection model, and CPR feedback of the patient is fed back in real time on the hemodynamic parameter, including CO, BVR, SV, etc. Human body key point localization of the operator and the hemodynamic parameter of the patient are combined as features, and instruction on CPR actions is output according to the pre-trained mapping model to guide the implementation of the CPR process.

The functions of the AI recognition module are therefore divided into an AI module 1 and an AI module 2.
The specific functions provided by the AI module 1 and the AI module 2 are shown in Table 1:

Table 1

| AI recognition target | AI recognition result | Corresponding model output | Voice guidance tactics Example | Screen display form | Response operation |
|---|---|---|---|---|---|
| Webcam position | Whether a webcam recording effect meets requirements of a video extraction | AI module 1: Background environment interference identifier | For example, "The webcam is too close to the rescuer, please replace it" | A distance measurement baseline between the webcam and the rescuer is displayed on the screen | The webcam is manually replaced according to the distance prompt in the screen, and the AI continues the detection |
| Scene environment | AI recognizes whether the surroundings of a first aid environment are dry and safe | AI module 1: Background environment interference identifier | For example, "A safety hazard is detected in the first aid environment, please clean the first aid scene" | A safety hazard obstacle obtained by image recognition is displayed on the screen and marked | The scene is cleaned up manually, and AI continues the detection |
| Patient pose | AI recognizes whether the pose of a patient lying on the ground to receive first aid meets the requirements | AI module 1: Classification categories of lying poses of the human body in the foreground | For example, "Make sure that the patient is lying flat and the head is tilted to one side" | A video of the correct and standard pose of the patient to receive first aid is displayed on the screen | A manual adjustment is performed, and AI continues the detection |
| Clothing state | AI recognizes whether a patient's clothing is open to reach first aid requirements | AI module 1: Foreground human body clothing identifier | For example, "Make sure that the patient's chest is not covered by clothing" | The area of a part of the patient's chest that needs to be exposed is displayed on the screen | A manual adjustment is performed, and AI continues the detection |
| Electrode plate placement position | AI recognizes whether the placement position of the rescuer's electrode plate is standard | AI module 1: Foreground human body key point position | For example, "The Electrode plate placement position is wrong, please adjust the position" | A correct position prompt diagram is displayed on the screen | A manual adjustment is performed, and AI continues the detection |

(continued)

| AI recognition target | AI recognition result | Corresponding model output | Voice guidance tactics Example | Screen display form | Response operation |
|---|---|---|---|---|---|
| Rescuer pose | AI recognizes whether the rescuer operation pose is standard | AI module 2: CPR implementer pose identifier | For example, "Ask the rescuer to keep the upper body leaning forward and push down vertically" | A standard operating animation of the rescuer is displayed on the screen | A manual adjustment is performed, and AI continues the detection |
| Press position | AI recognizes whether the CPR press position of the rescuer is standard | AI module 2: CPR press position identifier | For example, "The CPR press position is wrong, please repeat" | A correct position prompt diagram is displayed on the screen | A manual adjustment is performed, and AI continues the detection |
| Press pose | AI recognizes whether the CPR press pose of the rescuer is standard | AI module 2: CPR press pose identifier | For example, "The CPR press pose is wrong, please repeat" | A correct operation pose is displayed on the screen | A manual adjustment is performed, and AI continues the detection |
| Press depth | AI recognizes whether the CPR press depth of the rescuer is standard | AI module 2: CPR press depth numeric value and identifier | For example, "The CPR press depth is not qualified, please adjust the operation" | Correct press depth prompt information is displayed on the screen | A manual adjustment is performed, and AI continues the detection |
| Press frequency | AI recognizes whether the CPR press frequency of the rescuer is standard | AI module 2: CPR press frequency numeric value and identifier | For example, "The CPR press frequency is too slow, please adjust the operation" | Correct frequency prompt information is displayed on the screen | A manual adjustment is performed, and AI continues the detection |
| Number of presses | AI recognizes whether the number of CPR presses of the rescuer is standard | AI module 2: Numeric value and identifier of the number of presses | For example, "The number of CPR presses is not up to standard, please continue" | A prompt of a correct number of presses is displayed on the screen | A manual adjustment is performed, and AI continues the detection |

The technology of the AI module 1 in the AI recognition module is implemented by means of a foreground-background separation model.

The use of the AED during CPR requires standardization of the state of the patient, as well as the wearing of the device, and it is also necessary to ensure that the patient is protected from the influence of the external environment during electrode defibrillation. The foreground and background separation technology based on artificial intelligence images is used to separate the foreground human body and the background environment, and to judge the patient upper body human body outline represented by the foreground to locate the position of the key point of the human body, whether or not clothing is worn, the human body lying pose, and other information respectively. A matching calculation is performed on the patient human body represented by the background and the external environment in which the AED device is located to exclude interference from the incoming environment during defibrillation.

The assistance process of a CPR preparation stage based on the depth image foreground and background separation technology may refer to Table 2 as follows:

Table 2

| S210 | **X** = $x_1$, $x_2$,... $x_t$ represents collected video images during an observation window respectively. |
|---|---|
| S220 | Preprocessing such as (0, 255) greying and filtering is performed on each frame in **X** = $x_1$, $x_2$,... $x_t$. |
| S230 | An extraction of a foreground and a background is performed on **X** = $x_1$, $x_2$,... $x_t$ by using a low-rank decomposition theory, represented as a foreground outline **Az** = **Az**$_1$, **Az**$_2$,... **Az**$_t$ and a background **E** = **E**$_1$, **E**$_2$,... **E**$_t$. |
| S240 | Localization of the key point and a feature extraction are performed on a feature image representation **Z** = $z_1$, $z_2$,... $z_t$ of the human body outline by using a texture extraction and a human body point localization technology. |
| S250 | A background **E**$_t$ at each moment t is matched to an overall background **E** so as to judge whether there is interference in the background environment. |
| S260 | A foreground human body key point is located, clothing is determined, and a background environment interference judgment identifier is output. |

For S230 therein, the foreground and background are separated and extracted from the continuously collected video based on the low-rank decomposition theory as follows:

$$\mathbf{X} = \mathbf{Az} + \mathbf{E} \qquad\qquad \text{Formula (1)}$$

where X represents a matrix of collected video images, each image needs to be transformed into a (0, 255) greyscale matrix, and Az represents a foreground portion obtained after the low-rank decomposition, which is a product of a low-rank representation matrix and a low-rank coefficient matrix. The meanings of z and A are as follows respectively, z represents the low-rank representation matrix, which is used for representing a feature image in an image-video application, and in this scenario, the feature image is directly related to a localization point of the human body; and A represents the low-rank coefficient matrix, which is used for representing dynamic coefficient changes of the foreground. E represents a background portion described by the matrix of the collected video images minus a foreground human body outline.

Through the implementation of the above specific process before the CPR implementation, it is possible to accurately and efficiently calculate the environmental interference feedback identifier before the CPR implementation, and extract the patient human body position feature of the foreground human body, so as to provide help for the intelligent AED first aid assistance.

The realistically collected images and videos represent a streaming structure of a three-dimensional space. In order to further enhance nonlinear properties of the low-rank decomposition, formula (1) is nonlinearized, a Frobenius norm is used to constrain a low rank of the matrix, and a real-time matrix decomposition solution process is transformed into an extremum problem under the Frobenius norm to be solved:

$$\min_{\mathbf{A},\mathbf{z}} \|\mathbf{X} - \Pi(\mathbf{Az})\|_F + \|\mathbf{z}\|_F + \|\mathbf{A}\|_F \qquad\qquad \text{Formula (2)}$$

where $\Pi$ is represented as a nonlinear function. In general, a deep network is used for a nonlinear representation and solution of formula (2), and specifically a multilayer neural network performs low-rank decomposition modeling, as follows:

$$\min_{\mathbf{z},\Theta} \frac{1}{2n}\sum_{t=1}^{n}\left\|x_t - g^{(K+1)}(g^{(K)}(\dots g^{(1)}(\mathbf{z}_t, \Theta^{(1)}), \Theta^{(K)}), \Theta^{(K+1)})\right\| + \frac{\beta}{2n}\|\mathbf{Z}\|_F^2 + \frac{\lambda}{2}\sum_{j=1}^{K+1}\left\|\Theta^{(j)}\right\|_F^2$$

Formula (3)

where $x_t$ represents an image greyscale matrix at a certain moment t; and in a forward propagation process of the network, an establishment process of the human outline is $g^{(K+1)}(g^{(K)}(\dots g^{(1)}(\mathbf{z}_i, \Theta^{(1)}), \Theta^{(K)}), \Theta^{(K+1)})$. In this representation, $g^{(k)}(\cdot)$ represents an activation function of a single-layer neural network, k = 1,2,... K + 1 represents a neural network depth, and $\Theta^{(k)}$ represents a set of parameters of the single-layer neural network, as follows:

$$\Theta^{(k)} = \{W^{(k)}, b^{(k)}\} \qquad \text{Formula (4)}$$

where $W^{(k)}$, $b^{(k)}$ represent a weight and a bias parameter of the single-layer neural network respectively. Thus, the nonlinear representation of the single-layer neural network in formula (3) is specified as:

$$g^{(k)}(\mathbf{z}, \Theta^{(k)}) = g^{(k)}(W^{(k)}\mathbf{z} + \mathbf{b}) \qquad \text{Formula (5)}$$

In formula (2), in the forward propagation process of the network, a background residual at a certain moment t may be calculated as:

$$\mathbf{E_t} = x_t - g^{(K+1)}(g^{(K)}(\ldots g^{(1)}(\mathbf{z_t}, \Theta^{(1)}), \Theta^{(K)}), \Theta^{(K+1)}) \qquad \text{Formula (6)}$$

For step S240 therein, a scale-invariant feature transform (SIFT) computer vision algorithm model is used for detecting and describing a local feature in a video, and searching for an extremum point in a spatial scale, and its position, scale, and rotation invariant are extracted for representing key point results of a human body and device wear.

For S250 therein, an overall average background may be represented by an average of the overall background E, i.e., $\overline{\mathbf{E}}$, and whether there is interference at a current moment is judged by calculating a difference between the background Et at each moment t and the average background $\overline{\mathbf{E}}$, as follows:

$$\varepsilon = \mathbf{E_t} - \overline{\mathbf{E}} \qquad \text{Formula (7)}$$

If the difference $\varepsilon$ is larger than a threshold set by a system, it is judged that there is environmental interference in the background at this time, and otherwise it is judged that there is no environmental interference.

Through the implementation of the above specific process before the CPR implementation, it is possible to more accurately and efficiently calculate the environmental interference feedback identifier before the CPR implementation, and extract the patient human body position feature of the foreground human body, so as to provide greater help for the intelligent AED first aid assistance.

An iterative process of the algorithm for separating the foreground and the background by the low-rank model is mainly a process of updating $\varepsilon$ of a discrimination threshold according to the user's usage as follows.

1. In the actual process of use, the user obtains, by the model through the inference process of the following formula, results of foreground and background separation portions respectively as:

$$A\mathbf{z} = g^{(K+1)}(g^{(K)}(\ldots g^{(1)}(\mathbf{z_i}, \Theta^{(1)}), \Theta^{(K)}), \Theta^{(K+1)})$$

$$\mathbf{E} = \mathbf{X} - g^{(K+1)}(g^{(K)}(\ldots g^{(1)}(\mathbf{z_t}, \Theta^{(1)}), \Theta^{(K)}), \Theta^{(K+1)})$$

2. According to a system prompt, a video frame corresponding to an actual start operation of the user is $\widehat{X}_t$ .

3. According to a corresponding moment, the calculated current moment background $\widehat{\mathbf{E}}_t$ and the average background $\overline{\mathbf{E}}$ are respectively:

$$\widehat{\mathbf{E}}_t = \widehat{x}_t - g^{(K+1)}(g^{(K)}(\ldots g^{(1)}(\mathbf{z_t}, \Theta^{(1)}), \Theta^{(K)}), \Theta^{(K+1)})$$

$$\overline{\mathbf{E}} = \frac{1}{N-1}\sum_{i=t}^{N-1} \mathbf{E_i}$$

4. The discrimination threshold is updated according to a difference between the background at the current moment and the average background:

$$\hat{\boldsymbol{\varepsilon}} = \widehat{\mathbf{E}}_t - \overline{\mathbf{E}}$$

The technology of the AI module 2 in the AI recognition module is implemented in a mode that incorporates CPR guidance of a human body skin feedback optical model.

An existing assistance method for a CPR implementation stage mainly focuses on the use of an image recognition technology to recognize the pose of the implementer, which is used for solving the standardization of CPR implementation action requirements. For example, a human body pose key point detection technology is widely used in CPR intelligent image assistance applications, and widely used models include Pose Landmark Model for a human body key point localization and monitoring model, ResNet-18, ResNet-50 and other residual network models. These models are used for a human body key point feature extraction, extracting operator actions, etc.

However, the above methods ignore the CPR receiving body, that is, the patient's feedback under CPR, and improper or excessive CPR may lead to secondary injuries to the patient. As for the above problem, the scheme of the present application proposes to establish a skin reflection optical model based on the rPPG technology, which is be used to improve the current human body pose key point detection model by combining the feedback of the patient hemodynamic parameters.

The main process for the realization of the functions of the AI module 2 is divided into a training process and an online inference process respectively as follows.

The training process (i.e., training and establishing the S100 CPR mapping model) may refer to Table 3:

Table 3

| S110 | A set of RPC collected video data (including video images of an implementer and a patient, and CPR action standard feedback) is input. |
|---|---|
| S120 | Each frame of a video is collected for preprocessing such as (0, 255) greying and filtering. |
| S130 | A conventional technology model is used to perform implementer human body pose key point detection and localization. |
| S140 | An rPPG technology is used to establish an optical skin reflection model. |
| S150 | The optical skin reflection model is used to further calculate a patient hemodynamic parameter. |
| S160 | A mapping relationship is trained and established according to a CPR action standard identifier with a feature of detecting and positioning a key point of implementer's human body posture and a feature of patient hemodynamic parameter. |
| S170 | Model mapping parameters are output. |

The online inference process (i.e., S300 providing instruction and guidance on CPR actions during CPR implementation) may refer to Table 4:

Table 4

| S310 | Collected video images during an RPC real-time observation window are input. |
|---|---|
| S320 | Each frame of a video is collected for preprocessing such as (0, 255) greying and filtering. |
| S330 | A conventional technology model is used to perform implementer human body pose key point detection and localization. |
| S340 | An rPPG technology is used to establish an optical skin reflection model. |
| S350 | The optical skin reflection model is used to further calculate a patient hemodynamic parameter. |
| S360 | The trained model mapping parameters are used to calculate the CPR action standard feedback with the feature of detecting and positioning a key point of implementer's human body posture and the feature of patient hemodynamic parameter. |
| Output: | The CPR action standard feedback is output. |

For steps S130 and S330 in the training and online inference processes: The Pose Landmark Model is used to monitor and locate 33 key points on a body of an operator.

For steps S140 and S340 in the training and online inference processes: based on a remote photovoltaic volumetric pulse wave (rPPG), a subtle color change caused by a pulse on a surface of a human body skin is detected by using a multi-wavelength RGB camera in a webcam to realize non-contact monitoring of a human body cardiac activity and a hemodynamic parameter.

A principle of using the rPPG technology to build the skin reflection optical model is that: a spectrum and color channel sensitivity of a light source formed by diffuse reflection of the skin are received by the camera, and the color is skin deformation/specular reflection change caused by the operation of the RPC implementer and a subtle color change caused by a pulse. The color change, i.e., the skin diffuse reflection light spectrum, is absorbed and reflected by the epidermal skin tissue and internal bones to varying degrees, which may reflect the hemodynamic activity state of the human body, and the analysis of the received light by using an artificial intelligence method may obtain feedback on the human body hemodynamic parameter.

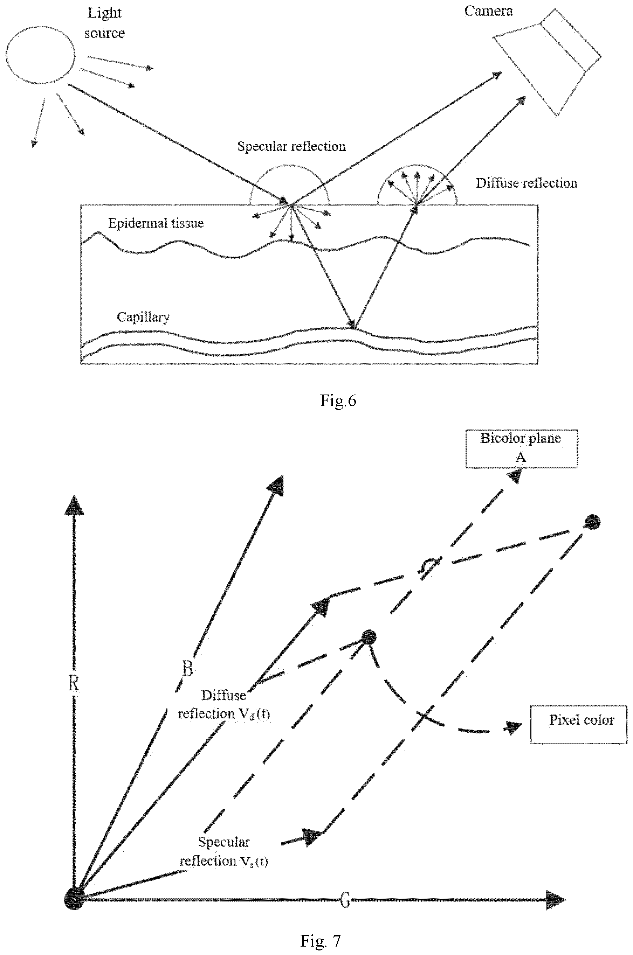

A schematic diagram of collecting rPPG signals by using the color camera may refer to Fig. 6, considering a light source illuminating a piece of human skin tissue containing pulsating blood and a remote color camera recording the image. The light source is assumed to have a constant spectral composition, but the intensity is variable (the intensity observed at the camera depends on a distance from the light source to the skin tissue and a camera sensor). The skin measured by the camera has a specific color (the skin color measured by the camera is a combination of the light source (e.g., intensity and spectrum), the intrinsic skin color, and the sensitivity of a camera color channel), and this color changes over time due to motion-induced changes in intensity/specular reflection and subtle color changes due to pulses. These temporal changes are proportional to a luminance intensity level.

Therefore, the skin reflection optical model in the present application is established by using a dichromatic reflection model, as shown in Fig. 7, where the specular reflection is specular-like light reflected from the skin surface without pulsation information. Therefore, its spectral composition is comparable to the spectral composition of the light source. A body motion affects the geometry between the light source, the skin surface and the camera, and has a time dependency. Diffuse reflection is related to the absorption and scattering of light in the skin tissue. The amount of hemoglobin and melanin in the skin tissue determines chroma.

Based on the dichromatic reflection model, the reflection of each skin pixel in the recorded image sequence may be defined as a time-varying function in a RGB channel:

$$\mathbf{C}_k(t) = I(t)\cdot(\mathbf{V}_s(t) + \mathbf{V}_d(t)) + \mathbf{V}_n(t) \qquad \text{Formula (8)}$$

where $C_k(t)$ represents a pixel value of a kth skin pixel at a moment t; I(t) represents a light intensity, which is determined by a luminance change of the light source itself as well as the distance between the light source, the skin tissue, and the camera, and which is modulated by two kinds of reflection, $V_s(t)$ and $V_d(t)$, where $V_s(t)$ represents specular reflection, which represents mirror-like reflected light emitted by the skin; $V_d(t)$ represents diffuse reflection, which represents light scattered and absorbed by the skin tissue; and $V_n(t)$ represents the quantization noise of the camera.

Specular reflection $V_s$ is light reflection from the skin surface, similar to a mirror. Since most of the light is reflected by the skin surface and only a small part of the light enters into the skin tissues, specular reflection is the main part of the two reflection components, and it does not contain any pulse information. However, since the deformation of the skin of the patient produced by the CPR operation leads to changes in the angle and distance between the light, the skin surface, and the camera, the specular reflection also contains a component of change, from which the specular reflection $\mathbf{V}_s$ may be defined as:

$$\mathbf{V}_s(t) = \mathbf{U}_s\cdot(s_0 + s(t)) \qquad \text{Formula (9)}$$

in the formula, $U_s$ represents a unit color vector of a specular reflection light spectrum captured by the camera, i.e., contribution degrees of red, green, and blue colors to the specular reflection light intensity; and $S_0$ and $s(t)$ represent light intensities of a direct current portion and an alternating current portion of the specular reflection light respectively.

Diffuse reflection $V_d$ relates to light absorbed and reflected by skin tissue and is a main component of a pulse signal. The effect of skin tissues on light reflection is mainly related to pigmentation of epidermal skin tissues, such as melanin, carotene, and the concentration of hemoglobin in the blood. Melanin, carotene, etc. affect the intrinsic reflection of the skin, which remains constant over time. Whereas the hemoglobin concentration changes periodically with cardiac activity, the intensity of light reflected through hemoglobin also produces periodic changes, thus reflecting pulse information. From this, the diffuse reflection $V_d(t)$ may be defined as:

$$\mathbf{V}_d(t) = \mathbf{U}_d \cdot d_0 + \mathbf{U}_p \cdot p(t) \qquad\qquad \text{Formula (10)}$$

in the formula, $U_d$ represents the unit color vector of the skin epidermal tissue, the direction of which is mainly determined by pigmentation and is related to the shade of skin color; and $d_0$ represents the intensity of the intrinsic diffuse reflection. Since blood absorbs light of different colors to different degrees, $U_p$ represents the contribution degrees of different color channels in the pulse signal, and $p(t)$ represents the intensity of the pulse signal.

It may be seen that the specular reflection $V_s$ and the diffuse reflection $V_d$ each contain a static portion that does not change with time and a dynamic portion that changes with time, and the static portions of the two kinds of reflection are combined and may be represented as:

$$\mathbf{U}_c \cdot c_0 = \mathbf{U}_s \cdot s_0 + \mathbf{U}_d \cdot d_0 \qquad\qquad \text{Formula (11)}$$

in the formula, $U_c$ represents a unit color vector of intrinsic reflection of the skin; and $c_0$ represents a light intensity of the intrinsic reflection.

Similarly, the light intensity $I(t)$ in formula (8) may also be divided into an intrinsic light intensity $I_0$ and a light intensity $I_0 \cdot i(t)$ that changes with time, and $I_0$ and $I_0 \cdot i(t)$ have the same direction and may be represented as:

$$I(t) = \mathbf{I}_0 \cdot (\mathbf{1} + i(t)) \qquad\qquad \text{Formula (12)}$$

formula (10) and (11) are substituted into the model represented by formula (8) to obtain the skin reflection model for the CPR patient as follows:

$$\mathbf{C}_k(t) = \mathbf{I}_0 \cdot (\mathbf{1} + i(t)) \cdot (\mathbf{U}_c \cdot c_0 + \mathbf{U}_s \cdot s(t) + p(t)) + \mathbf{V}_n(t) \qquad\qquad \text{Formula (13)}$$

By most of existing rPPG methods, a time domain signal is formed by performing space averaging on a pixel point in each frame of ROI region, and space averaging of enough pixel points can effectively reduce the quantization noise of the camera (i.e. $V_n(t)$ may be ignored). At the same time, intensities of alternating current components are very small compared to a direct current component captured by the camera, and thus product terms of the alternating current components, such as $i(t) \cdot s(t)$, $i(t) \cdot p(t)$, etc., may be ignored. Considering the above two points and expanding formula (13) can obtain:

$$\mathbf{C}_k(t) \approx \mathbf{I}_0 \cdot \mathbf{U}_c \cdot c_0 + \mathbf{I}_0 \cdot \mathbf{U}_c \cdot c_0 \cdot i(t) + \mathbf{I}_0 \cdot \mathbf{U}_s \cdot s(t) + \mathbf{I}_0 \cdot \mathbf{U}_p \cdot p(t) \qquad \text{Formula (14)}$$

Under the skin reflection optical model, an observation value $C_k(t)$ becomes a linear mixture of one direct current component and three alternating current source signals $i(t)$, $s(t)$, and $p(t)$, which are respectively: $I_0 \cdot U_c \cdot c_0$ representing the direct current component, $I_0 \cdot U_c \cdot c_0 \cdot i(t)$ representing the light intensity alternating current component, $I_0 \cdot U_s \cdot s(t)$ representing the specular reflection component, and $I_0 \cdot U_p \cdot p(t)$ representing the pulse signal component.

For steps S150 and S350 in the training and online inference processes: weighted averaging is firstly performed on k skin pixels in formula (14) to calculate a one-dimensional pulse signal of the rPPG:

$$C(t) \approx \sum_{k=1}^{K} \mathbf{C}_k(t) \qquad\qquad \text{Formula (15)}$$

From formula (14), it may be seen that $C(t)$ is a time-dependent one-dimensional signal, which mainly contains two portions of information, one portion comes from illumination and light source influences, including $I_0 \cdot U_c \cdot c_0$, $I_0 \cdot U_c \cdot c_0 \cdot i(t)$, and $I_0 \cdot U_s \cdot s(t)$. The other portion comes from a pulse separation signal $I_0 \cdot U_p \cdot p(t)$. The pulse component signal shows a blood supply process of ventricles, where the periodic systolic-diastolic activity of the heart causes blood to be shot from the heart into arteries and then returned to the heart by veins, forming an organic cycle. A pulse wave is formed by the heart's beat propagating peripherally along arterial vessels and blood flow, and the diastole and systole of the ventricles correspond to a diastolic peak and a systolic peak of the signal. Thus, $C(t)$ is directly related to hemodynamic parameters. Typical hemodynamic parameters may be calculated by the following method.

Heart rate: a highest peak value of the signal represents the absorption peak of the one-dimensional pulse signal in formula (15) and corresponds to the systolic process of the ventricle. Similar to the mode the RRI is calculated for ECG signals, calculating the time interval between two absorption peaks (PPI) allows the heart rate to be calculated.

HRV: heart rate variability (HRV) is a small difference in time between consecutive sinus cardiac cycles. Under physiologic conditions, HRV is produced primarily as a result of a general difference of several tens of milliseconds in cardiac per-stroke intervals produced by cardiac sinus node autoregulatory activity. HRV during CPR directly reflects changes in pressure reflexes of the patient and the adjustment of a respiratory activity. The number of times a pulse wave beats per minute (i.e. pulse rate) and the heart rate are consistent. Therefore, the peak-to-peak interval (PPI) in the pulse wave signal may be used for an HRV calculation instead of the R-R interval (RRI) in an electrocardiosignal.

SV: a stroke volume refers to the amount of blood ejected from a ventricle on one side during a single heart stroke, referred to as stroke volume, and is directly related to the press exerted by the operator during CPR. A pulse tour analysis method is used to calculate the pulse stroke volume as follows:

$$\text{SV} \approx \int d\text{C}'(\text{t})/d\text{t} \qquad\qquad \text{Formula (16)}$$

In the above formula, C'(t) represents a complete cycle of the C(t) signal. The stroke volume (SV) is estimated according to a signal change integral from end diastole to end systole, i.e., a systolic portion of the C(t) signal after a systolic peak, up to a process of aortic valve closure.

CO: CO refers to the amount of blood pumped per minute by a left or right ventricle, i.e. a product of the stroke volume and the heart rate, and has important monitoring significance during CPR.

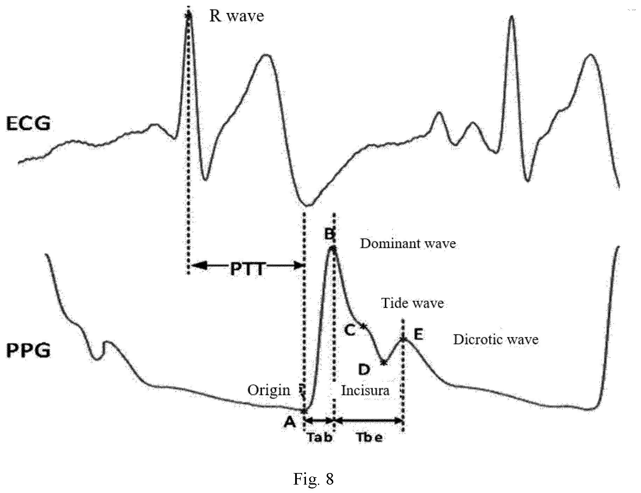

Referring to ECG and PPG oscillograms shown in Fig. 8, a peak value (R wave group) of an electrocardiogram (ECG) comes from ventricular systole, and a peak value (dominant wave) of a PPG comes from vasoconstriction, wherein the transmission time for blood to reach a measurement position after it is delivered from the heart is reflected by pulse transit time (PTT). The heart rate variability (HRV) may be calculated by calculating an interval between R waves of the ECG signal (RRI, R-R Interval) and also by calculating an interval between main peaks of the PPG.

For steps S160 and S360 in the training and online inference processes: the process of guiding CPR action norms according to the rescuing action of the implementer and the blood flow state feedback of the patient is the process of modeling the mapping relationship with the implementer human body key-point localization and the patient hemodynamic parameter as features, and with the CPR action implementation norm identifier as the feedback, as follows:

$$\mathbf{Y} = \text{f}(\mathbf{X})$$

$$\mathbf{X} = \dot{\mathbf{X}} \oplus \ddot{\mathbf{X}} \qquad\qquad \text{Formula (17)}$$

where the input feature X consists of two sets of features $\dot{\mathbf{X}}$ and $\ddot{\mathbf{X}}$, and $\oplus$ represents splicing of matrix vectors. $\dot{\mathbf{X}}$ and $\ddot{\mathbf{X}}$ represent an implementer human body key point localization point feature, and a feature of patient hemodynamic parameter respectively. Y represents a numerical identifier of the action implementation norms of CPR.

The mapping relationship may be determined by means of a large number of experiments combined with observations and statistics and may also be obtained by using machine learning and deep learning methods for a calculation, and the CPR action norm identifier for training needs to be marked manually.

In the following, the above mapping relationship is established exemplarily through a multiple regression model:

$$\mathbf{Y} = \mathbf{W} \cdot \mathbf{X} + \mathbf{b}$$

$$\mathbf{X} = \dot{\mathbf{X}} \oplus \ddot{\mathbf{X}} \qquad\qquad \text{Formula (18)}$$

In the above formula, W and b represent parameter weights of the model respectively. In the embodiment of the present application, model parameters mainly include:

in the training process, training is performed to obtain the model parameters W and b mainly by using X calculated in steps S130 to S150, and the CPR action standard identifier Y contained in the training dataset; and
in the real-time inference process, the CPR action standard identifier Y is calculated through formula (18) with the feature set X representing the rescuing action of the implementer and the blood flow state feedback of the patient obtained by real-time calculations from step S330 to step S350, as well as the trained model parameters W and b.

Meanwhile, the present application further proposes an iterative scheme for the algorithm of the CPR mapping relationship model, and an iterative process of the algorithm for the CPR mapping relationship model is as follows.

1. In the actual process of use, the user obtains, by the model through the inference process of the following formula, the CPR action standard identifier Y:

$$Y = W \cdot X + b$$

$$X = \dot{X} \oplus \ddot{X}.$$

2. The actual operation performed by the user according to system prompts is transformed into a numeric identifier $\hat{Y}$.
3. The model takes $\hat{Y}$ as a target output and takes the collected image data X as a model input to update the model parameters.
4. A model parameter updating problem is transformed into a least squares problem for solving, and the specific model is updated by updating the original model parameters W and b through the following formula.

$$\min_{W,b} \left(\hat{Y} - W \cdot X + b\right)^{T} \left(\hat{Y} - W \cdot X + b\right)$$

where $(\cdot)^{T}$ is a transpose operation computation of the matrix.

Through the iteration of the algorithm for separating the foreground and background and the iteration of the algorithm for the CPR mapping relationship model, it is possible to further iteratively update the relevant model algorithm with the data collected during the actual use of the intelligent AED apparatus, and the corresponding actions performed by the implementer, so as to make the intelligent guidance function of the intelligent AED apparatus be more closely matched with the real operation of the implementer of the actual operation, and also to allow the relevant model algorithm itself to be updated constantly and be more intelligent as the scheme of the present application is promoted and used.

By the implementation of the above embodiments, the purpose of the present application is reached, which combines deep learning, constructions of important models before and after the CPR implementation, and intelligent AED first aid assistance in which a full first aid process is accompanied by artificial intelligence-assisted guidance, so that the requirements of professionalism for the implementer in an emergency situation can be reduced. The implementer who has undergone a low frequency of basic life support training can also quickly perform CPR first aid on the patient, thus ensuring that the CPR first aid can be performed quickly and effectively on the patient in a sudden state and without professional staff to increase a patient survival rate and improve a first aid effect. Accurate and complete treatment information is also provided for subsequent treatment, so as to further ensure the life safety and health protection of the patient.

The above embodiments only serve to illustrate technical concepts and features of the present application, are intended to enable person familiar with the technology to understand the contents of the present application and to implement them accordingly, and cannot be used to limit the scope of protection of the present application. Any equivalent changes or modifications made according to the spirit essence of the present application shall be covered by the scope of protection of the present application.

**Claims**

1. An intelligent AED first aid assistance method, comprising the following steps:

S100, training and establishing a CPR mapping model,
specifically, training and establishing a CPR mapping relationship, according to a CPR action standard identifier, with a feature of detecting and positioning a key point of implementer's human body posture and a feature of patient hemodynamic parameter, and outputting the CPR mapping model;
S200, before CPR implementation, recognizing whether a first aid condition meets a standard,
specifically, after an intelligent AED apparatus is enabled, performing a separation of a foreground and a background before the CPR implementation, recognizing and judging whether a current first aid environment meets the first aid standard with respect to the environmental background, and recognizing and judging whether a patient human body position feature meets the first aid standard with respect to the human body foreground;

S300, during the CPR implementation, providing instruction and guidance on CPR actions,

specifically, during the CPR implementation, using a human body pose recognition model to position a key point of an implementer and obtain the feature for detecting and positioning the key point of implementer's human body posture, establishing a human body skin reflection model, feeding CPR feedback of a patient back in real time on the feature of patient hemodynamic parameter, and in combination with the feature of detecting and positioning a key point of implementer's human body posture and the feature of patient hemodynamic parameter, using the trained CPR mapping model to output CPR action standard feedback so as to perform instruction on CPR actions and to guide implementation of a CPR process.

2. The intelligent AED first aid assistance method according to claim 1, further **characterized by** comprising starting a remote AI first aid function after the intelligent AED apparatus is enabled, connecting the intelligent AED apparatus to a remote doctor through a first aid platform server, and uploading foreground data and background data before the CPR implementation as well as implementer data and patient data during the CPR implementation to the first aid platform server, so that the remote doctor participates in CPR first aid with audio and a video in a whole process before and during the CPR implementation.

3. The intelligent AED first aid assistance method according to claim 2, **characterized in that** AED data collection is automatically awakened after the intelligent AED apparatus is enabled, an AI identification of the foreground data, the background data, the implementer data, and the patient data is performed, an identification output is performed according to AI identification results corresponding to the CPR mapping model, and voice guidance and a screen display are provided in performing the instruction on CPR actions.

4. The intelligent AED first aid assistance method according to claim 1, **characterized in that** step S200 of recognizing whether the first aid condition meets the standard before the CPR implementation comprises the following steps:

S210, collecting video images before the CPR implementation,
specifically, collecting the video image of an environment in which the AED device is located and the video image of a patient human body before the CPR implementation;
S220, preprocessing the video images before the CPR implementation,
specifically, performing greying and filtering preprocessing on the collected video image of the environment in which the AED device is located and the video image of the patient human body;
S230, performing the separation of the foreground and the background before the CPR implementation,
specifically, separating the environmental background from the foreground human body;
S240: judging whether the current first aid environment meets the first aid standard,
specifically, calculating an environmental interference feedback identifier before the CPR implementation according to the environmental background, and judging whether the environmental background meets the first aid standard;
S250, judging whether the patient human body position feature meets the first aid standard,
specifically, extracting the patient human body position feature of the foreground human body, and judging whether the patient meets the first aid standard according to the patient human body position feature; and
S260, outputting judgment identifiers of the foreground and the background before the CPR implementation.

5. The intelligent AED first aid assistance method according to claim 4, **characterized in that**

in step S230 of performing the separation of the foreground and the background before the CPR implementation, the separation of the foreground and the background is performed by using a deep low-rank model; the background is extracted with a residual component, and background residual changes during defibrillation are compared; and the patient human body position feature is automatically traced with a low-rank component;
in step S240 of judging whether the current first aid environment meets the first aid standard, whether an output photography distance is suitable and whether the environment is safe and dry are judged according to the environmental interference feedback identifier; and
in step S250 of judging whether the patient human body position feature meets the first aid standard, whether a patient pose meets a requirement, whether clothing meets a requirement, and whether an electrode plate placing position is correct are judged according to the patient human body position feature.

6. The intelligent AED first aid assistance method according to claim 4, **characterized in that**

in step S210 of collecting the video images before the CPR implementation, $X = x_1, x_2, \ldots x_t$ is input, where X represents a matrix of the collected video images;

in step S220 of preprocessing the video images before the CPR implementation, each frame in $X = x_1, x_2, \ldots x_t$ is transformed into a (0, 255) greyscale matrix, and filtering preprocessing is performed;

in step S230 of performing the separation of the foreground and the background before the CPR implementation, $\mathbf{X} = x_1, x_2, \ldots x_t$ is subjected to a foreground and background extraction by using a low-rank decomposition theory, $\mathbf{X} = \mathbf{Az} + \mathbf{E}$; Az represents a foreground portion obtained after low-rank decomposition, the foreground portion $\mathbf{Az} = \mathbf{Az}_1, \mathbf{Az}_2, \ldots \mathbf{Az}_t$; and E represents a background portion described by the matrix of the collected video images minus a foreground human body outline, the background portion $\mathbf{E} = \mathbf{E}_1, \mathbf{E}_2, \ldots \mathbf{Et}$, the background is extracted with a residual component, and background residual changes during defibrillation are compared;

in step S240 of judging whether the current first aid environment meets the first aid standard, a background $E_t$ at each moment t is matched to the overall background E so as to judge whether there is interference in the background environment;

in step S250 of judging whether the patient human body position feature meets the first aid standard, localization of the key point and a feature extraction are performed on a feature image representation $\mathbf{Z} = z_1, z_2, \ldots z_t$ of the human body outline by using a texture extraction and a human body point localization technology; and

in step S260 of outputting the judgment identifiers of the foreground and the background before the CPR implementation, foreground human body key point localization is output, clothing is determined, and a background environment interference judgment identifier is output.

7. The intelligent AED first aid assistance method according to claim 6, **characterized in that**

in step S230 of performing the separation of the foreground and the background before the CPR implementation, the formula $\mathbf{X} = \mathbf{Az} + \mathbf{E}$ is nonlinearized, a Frobenius norm is used to constrain a low rank of the matrix, and a real-time matrix decomposition solution process is transformed into an extremum problem under the Frobenius norm to be solved:

$$\min_{\mathbf{A,z}} \|\mathbf{X} - \Pi(\mathbf{Az})\|_F + \|\mathbf{z}\|_F + \|\mathbf{A}\|_F,$$

where $\Pi$ is represented as a nonlinear function;

a deep network is then used for a nonlinear representation and solution of the above formula, and specifically a multilayer neural network performs low-rank decomposition modeling, as follows:

$$\min_{\mathbf{z,\Theta}} \frac{1}{2n} \sum_{t=1}^{n} \left\| x_t - g^{(K+1)}(g^{(K)}(\ldots g^{(1)}(\mathbf{z}_t, \Theta^{(1)}), \Theta^{(K)}), \Theta^{(K+1)}) \right\| + \frac{\beta}{2n} \|\mathbf{Z}\|_F^2 + \frac{\lambda}{2} \sum_{j=1}^{K+1} \left\| \Theta^{(j)} \right\|_F^2,$$

where $x_t$ represents an image greyscale matrix at a certain moment t;

in a forward propagation process of the multilayer neural network, an establishment process of the human outline is $g^{(K+1)}(g^{(K)}(\ldots g^{(1)}(\mathbf{z}_i, \Theta^{(1)}), \Theta^{(K)}), \Theta^{(K+1)})$, where $g^{(k)}(\cdot)$ represents an activation function of a single-layer neural network, k = 1,2,... K + 1 represents a neural network depth, and $\Theta^{(k)}$ represents a set of parameters of the single-layer neural network, as follows:

$$\Theta^{(k)} = \{W^{(k)}, b^{(k)}\},$$

where $W^{(k)}$ represents a weight of the single-layer neural network, $b^{(k)}$ represents a bias parameter of the single-layer neural network, and a nonlinear representation of the single-layer neural network in the multilayer neural network is specifically: $g^{(k)}(\mathbf{z}, \Theta^{(k)}) = g^{(k)}(W^{(k)}\mathbf{z} + \mathbf{b})$;

in the forward propagation process of the multilayer neural network, a background residual at a certain moment t may be calculated as: $\mathbf{E}_t = xt - g^{(K+1)}(g^{(K)}(\ldots g^{(1)}(\mathbf{z}_t, \Theta^{(1)}), \Theta^{(K)}), \Theta^{(K+1)})$;

in step S240 of judging whether the current first aid environment meets the first aid standard, an overall average background is represented by an average of the overall background E, i.e., an average background $\overline{\mathbf{E}}$, and whether there is interference at a current moment is judged by calculating a difference between the background $E_t$ at each moment t and the average background $\overline{\mathbf{E}}$, as follows:

$$\varepsilon = \mathbf{E}_t - \overline{\mathbf{E}},$$

if the difference ε is larger than a threshold set by a system, it is judged that there is environmental interference in the background at this time, and otherwise it is judged that there is no environmental interference; and

in step S250 of judging whether the patient human body position feature meets the first aid standard, a computer vision algorithm model of scale-invariant feature transform is used to detect and describe a local feature in a video, an extremum point is searched for in a spatial scale; and a position, a scale, and a rotation invariant thereof are extracted for representing key point results of the human body and device wear, so as to obtain the patient human body position feature.

8. The intelligent AED first aid assistance method according to claim 7, **characterized in that** in step S240 of judging whether the current first aid environment meets the first aid standard, a process of updating a discrimination threshold $\hat{\varepsilon}$ according to a use of the intelligent AED apparatus is as follows:

obtaining, by a deep low-rank model through a following inference process, a result of the foreground human body portion as:

$$A\mathbf{z} = g^{(K+1)}(g^{(K)}(\ldots g^{(1)}(\mathbf{z}_i, \Theta^{(1)}), \Theta^{(K)}), \Theta^{(K+1)});$$

obtaining, by the deep low-rank model through the following inference process, a result of the environmental background portion as:

$$\mathbf{E} = \mathbf{X} - g^{(K+1)}(g^{(K)}(\ldots g^{(1)}(\mathbf{z}_t, \Theta^{(1)}), \Theta^{(K)}), \Theta^{(K+1)});$$

obtaining a video frame $\widehat{X}_t$ corresponding to an actual start operation of the implementer;

calculating a current moment background $\widehat{\mathbf{E}}_t$ according to a moment corresponding to the video frame:

$$\widehat{\mathbf{E}}_t = \widehat{x}_t - g^{(K+1)}(g^{(K)}(\ldots g^{(1)}(\mathbf{z}_t, \Theta^{(1)}), \Theta^{(K)}), \Theta^{(K+1)});$$

calculating the average background $\overline{\mathbf{E}}$:

$$\overline{\mathbf{E}} = \frac{1}{N-1}\sum_{i=t}^{N-1} \mathbf{E}_i;$$

and

updating the discrimination threshold according to the current moment background $\widehat{\mathbf{E}}_t$ and the average background $\overline{\mathbf{E}}$:

$$\hat{\boldsymbol{\varepsilon}} = \widehat{\mathbf{E}}_t - \overline{\mathbf{E}}.$$

9. The intelligent AED first aid assistance method according to claim 1, **characterized in that** step S100 of training and establishing the CPR mapping model comprises the following steps:

S110, collecting a training data set, collecting video images of the implementer and the patient during training, and gathering the CPR action standard identifier;
S120, preprocessing the training video images,
specifically, performing greying and filtering preprocessing on the collected video image;
S130, obtaining the feature of detecting and positioning a key point of implementer's human body posture during training,
specifically, detecting and positioning the key point of the implementer's human body posture, and obtaining the feature of detecting and positioning the key point of implementer's human body posture;
S140, establishing the human body skin reflection model of the patient during training,
specifically, using an optical rPPG technology to establish the human body skin reflection model during training;
S150, obtaining the feature of patient hemodynamic parameter during training,
specifically, using the human body skin reflection model to further calculate and obtain the feature of patient hemodynamic parameter during training;

S160, training and establishing the CPR mapping relationship,

specifically, training and establishing the CPR mapping relationship, according to the CPR action standard identifier, with the feature of detecting and positioning a key point of implementer's human body posture and the feature of patient hemodynamic parameter during training; and

S 170, outputting a parameter of the CPR mapping model,

specifically, outputting the parameter of the CPR mapping model according to the CPR mapping relationship.

10. The intelligent AED first aid assistance method according to claim 9, **characterized in that** step S300 of providing the instruction and guidance on CPR actions during the CPR implementation comprises the following steps:

S310, collecting the video images during the CPR implementation,

specifically, collecting the video image of the implementer and the video image of the patient during the CPR implementation;

S320, preprocessing the video images during the CPR implementation,

specifically, performing greying and filtering preprocessing on the collected video image of the implementer and the video image of the patient;

S330, obtaining the feature of detecting and positioning a key point of implementer's human body posture during the CPR implementation,

specifically, detecting and positioning the key point of the implementer's human body posture, and obtaining the feature of detecting and positioning the key point of implementer's human body posture during the CPR implementation;

S340, establishing the human body skin reflection model of the patient during the CPR implementation,

specifically, using the optical rPPG technology to establish the human body skin reflection model during the CPR implementation;

S350, obtaining the feature of patient hemodynamic parameter during the CPR implementation,

specifically, using the human body skin reflection model to further calculate and obtain the feature of patient hemodynamic parameter during the CPR implementation;

S360, calculating the CPR action standard feedback,

specifically, using the trained CPR mapping model to calculate the CPR action standard feedback, with the feature of detecting and positioning the key point of implementer's human body posture and the feature of patient hemodynamic parameter during the CPR implementation; and

S370, outputting the CPR action standard feedback, feeding back a CPR action standard to the implementer who is implementing CPR, performing the instruction on CPR actions, and guiding the implementation of the CPR process.

11. The intelligent AED first aid assistance method according to claim 10, **characterized in that** for establishment of the human body skin reflection model, based on a remote photovoltaic volumetric pulse wave, a subtle color change caused by a pulse on a surface of a human body skin is detected by using a multi-wavelength RGB camera in a webcam to realize non-contact monitoring of a human body cardiac activity and a hemodynamic parameter so as to establish the human body skin reflection model.

12. The intelligent AED first aid assistance method according to claim 11, **characterized in that** for the establishment of the human body skin reflection model, firstly, based on a dichromatic reflection model, reflection of each skin pixel in a recorded image sequence is defined as a time-varying function: $\mathbf{C}_k(t) = I(t) \cdot (\mathbf{V}_s(t) + \mathbf{V}_d(t)) + \mathbf{V}_n(t)$ in a RGB channel, where $C_k(t)$ represents a pixel value of a $k^{th}$ skin pixel at a moment t, I(t) represents a light intensity, $V_s(t)$ represents specular reflection, $V_d(t)$ represents diffuse reflection, and $V_n(t)$ represents a quantization noise of the camera;

the specular reflection $V_s(t)$ is defined as: $\mathbf{V}_s(t) = \mathbf{U}_s \cdot (s_0 + s(t))$, where $U_s$ represents a unit color vector of a spectrum of specular reflection light captured by the camera, $S_0$ represents a light intensity of a direct current portion of the specular reflection, and s(t) represents a light intensity of an alternating current portion of the specular reflection;

the diffuse reflection $V_d(t)$ is defined as: $\mathbf{V}_d(t) = \mathbf{U}_d \cdot d_0 + \mathbf{U}_p \cdot p(t)$, where $U_d$ represents a unit color vector of skin epidermal tissue, $d_0$ represents an intensity of intrinsic diffuse reflection, $U_p$ represents a contribution degree of different color channels in a pulse signal, and p(t) represents an intensity of the pulse signal;

the specular reflection $V_s$ and the diffuse reflection $V_d$ each contain a static portion that does not change with time and a dynamic portion that changes with time, and the static portions of the two kinds of reflection are combined and represented as: $\mathbf{U}_c \cdot c_0 = \mathbf{U}_s \cdot s_0 + \mathbf{U}_d \cdot d_0$, where $U_c$ represents a unit color vector of intrinsic reflection of the skin; and $c_0$ represents a light intensity of the intrinsic reflection;

the light intensity I(t) is also divided into an intrinsic light intensity $I_0$ and a light intensity $I_0$ i(t) that changes with time, and $I_0$ and $I_0$ i(t) have the same direction, represented as: I(t) = $I_0$·(**1** + i(t)); and

thus, the human body skin reflection model is obtained as follows: $\mathbf{C}_k(t) = \mathbf{I}_0·(\mathbf{1} + i(t))·(\mathbf{U}_c·c_0 + \mathbf{U}_s·s(t) + p(t)) + \mathbf{V}_n(t)$.

13. The intelligent AED first aid assistance method according to claim 12, **characterized in that** a time domain signal is formed by performing space averaging on a pixel point in each frame of ROI region, space averaging of enough pixel points is obtained to effectively reduce the quantization noise of the camera, so as to ignore the quantization noise $V_n$(t) of the camera, at the same time, intensities of alternating current components are very small compared to a direct current component captured by the camera, and a product term of the alternating current components is also ignored, so as to unfold the human body skin reflection model to obtain:

$$\mathbf{C}_k(t) \approx \mathbf{I}_0·\mathbf{U}_c·c_0 + \mathbf{I}_0·\mathbf{U}_c·c_0·i(t) + \mathbf{I}_0·\mathbf{U}_s·s(t) + \mathbf{I}_0·\mathbf{U}_p·p(t);$$

and

under the unfolded human body skin reflection optical model, an observation value $C_k$(t) becomes a linear mixture of the one direct current component and three alternating current source signals i(t), s(t), and p(t), which are $I_0·U_c·c_o$ representing the direct current component, $I_0·U_c·c_0·i(t)$ representing the light intensity alternating current component, $I_0·U_s·s(t)$ representing the specular reflection component, and $I_0·U_p·p(t)$ representing the pulse signal component, respectively.

14. The intelligent AED first aid assistance method according to claim 13, **characterized in that** in the step of obtaining the feature of patient hemodynamic parameter, firstly, weighted averaging is performed on k skin pixels in the unfolded human body skin reflection model to calculate a one-dimensional pulse signal: $C(t) \approx \sum_{k=1}^{K} \mathbf{C}_k(t)$ of the remote photovoltaic volumetric pulse wave, and a calculation of each feature of patient hemodynamic parameter comprises:

a heart rate: a highest peak value of the one-dimensional pulse signal represents an absorption peak of the signal, a time interval between two absorption peaks in the one-dimensional pulse signal is a peak-to-peak interval (PPI) in the pulse wave signal, and the peak-to-peak interval (PPI) in the pulse wave signal is calculated so as to calculate the heart rate;

HRV: HRV represents heart rate variability and is calculated by replacing an RRI in an electrocardiosignal with the peak-to-peak interval (PPI) in the pulse wave signal;

SV: SV represents a stroke volume, a pulse tour analysis method is used to calculate the pulse stroke volume, SV $\approx \int$ dC'(t)/dt, where C'(t) represents a complete cycle of the C(t) signal; and

CO: CO represents a cardiac output pumped per minute by a left or right ventricle and is a product of the stroke volume and the heart rate.

15. The intelligent AED first aid assistance method according to claim 14, **characterized in that** the step of training and establishing the CPR mapping relationship or the step of calculating the CPR action standard feedback is a process of the CPR mapping relationship that combines the feature of detecting and positioning the key point of implementer's human body posture and the feature of patient hemodynamic parameter, with the CPR action standard as the feedback, the process is as follows:

inputting the features:

$$\mathbf{Y} = f(\mathbf{X});$$

and

$$\mathbf{X} = \dot{\mathbf{X}} \oplus \ddot{\mathbf{X}};$$

where a feature set X consists of two sets of features $\dot{\mathbf{X}}$ and $\ddot{\mathbf{X}}$, $\oplus$ represents splicing of matrix vectors, $\dot{\mathbf{X}}$ represents the feature of detecting and positioning the key point of implementer's human body posture, $\ddot{\mathbf{X}}$ represents the feature of patient hemodynamic parameter, and Y represents the CPR action standard identifier;

the CPR mapping relationship is established by means of a large number of experiments combined with observations and statistical decisions, or is established by using machine learning and deep learning methods for a calculation, wherein the CPR action standard identifier Y for training is marked manually, and a training dataset is established;

the CPR mapping relationship is then established through a multiple regression model:

$$\mathbf{Y} = \mathbf{W}{\cdot}\mathbf{X} + \mathbf{b};$$

$$\mathbf{X} = \dot{\mathbf{X}} \oplus \ddot{\mathbf{X}};$$

in the above formula, W represents a feature weight of the CPR mapping model, and b represents a bias term of the CPR mapping model;

in step S100 of training and establishing the CPR mapping model, training is performed to obtain model parameters W and b according to the calculated feature set X, and the CPR action standard identifier Y contained in the training dataset; and

in step S300 of providing the instruction and guidance on CPR actions during the CPR implementation, the CPR action standard identifier Y is calculated through the feature set X obtained from the real-time calculation, and the trained model parameters W and b.

16. The intelligent AED first aid assistance method according to claim 15, **characterized in that** in step S360 of calculating the CPR action standard feedback, the model parameters W and b are updated according to the use of the intelligent AED apparatus in the following process:

obtaining the CPR action standard identifier Y;

obtaining and transforming an operation actually performed by the implementer according to the instruction on CPR actions into a CPR numeric identifier $\hat{\mathbf{Y}}$;

updating the model parameters W and b with the CPR numeric identifier $\hat{\mathbf{Y}}$ as a target output and the collected feature set X as a model input; and

updating the original model parameters W and b in the following mode:

$$\min_{\mathbf{W},\mathbf{b}} \left(\widehat{\mathbf{Y}} - \mathbf{W}{\cdot}\mathbf{X} + \mathbf{b}\right)^{\mathrm{T}}\left(\widehat{\mathbf{Y}} - \mathbf{W}{\cdot}\mathbf{X} + \mathbf{b}\right),$$

where $(\cdot)^{\mathrm{T}}$ is a transpose operation computation of the matrix.

17. An intelligent AED first aid assistance system used for intelligent AED first aid assistance in the method according to any one of claims 1 to 16, comprising: an intelligent AED apparatus, a first aid platform server, and a first aid medical terminal; wherein

the intelligent AED apparatus is an apparatus for external defibrillation with an AI recognition module, a CPR mapping model, which is output after training and establishing a CPR mapping relationship according to a CPR action standard identifier by combining a feature of detecting and positioning the key point of implementer's human body posture and a feature of patient hemodynamic parameter, is deployed in the AI recognition module, and the AI recognition module is configured to recognize whether a first aid condition meets a standard before CPR implementation, and provide instruction and guidance on CPR actions during the CPR implementation;

the first aid platform server communicates with the intelligent AED apparatus in a long-distance wireless mode, and the first aid platform server is used for receiving data uploaded by the intelligent AED apparatus, storing, forwarding and sending data required by a first aid doctor to the first aid medical terminal; and

the first aid medical terminal is used for displaying the received data and performing a real-time audio and video call with the intelligent AED apparatus.

18. An intelligent AED first aid assistance apparatus used for intelligent AED first aid assistance in the method according to any one of claims 1 to 16, comprising: a master control module, an awakening detection module, a video collection module, an audio collection module, a power module, a communication module, an AI recognition module, a storage module, a voice broadcasting module, a video display module, and an early warning module; wherein

the master control module is configured for information centralization, storage, analysis and decision making;

the awakening detection module is configured to automatically awaken an AED data collection when the intelligent AED apparatus is used;

the audio collection module is configured to collect real-time scene audio;

the video collection module is configured to collect a real-time scene video;

the power module is configured to supply power to other modules;

the AI recognition module is configured to recognize an electrode plate installation position and a first aid environment interference factor, perform a CPR quality analysis, recognize physiological data of a patient, and output error correction result information;

the storage module is configured to store AED running information, treatment information, detection information, and AI recognition feedback information;

the communication module is responsible for communicating with the first aid platform server;

the voice broadcasting module is configured to broadcast a voice prompt and audio of a video call;

the video display module is configured to display a video prompt and a video of a video call; and

the early warning module is configured to be responsible for warning when the first aid medical terminal does not respond to a first aid event.

19. A readable storage medium, storing a control program thereon, **characterized in that** the control program, when executed by an AI recognition module, causes the AI recognition module to execute the steps of the method according to any one of claims 1 to 16.

S100 Training and establishing a CPR mapping model

Training and establishing a CPR mapping relationship according to a CPR action standard identifier, with a feature of detecting and positioning a key point of implementer's human body posture and a feature of patient hemodynamic parameter, and outputting the CPR mapping model

S200 Recognizing whether a first aid condition meets a standard before CPR implementation

Performing, after an intelligent AED apparatus is enabled, a separation of a foreground and a background before the CPR implementation, recognizing and judging whether a current first aid environment meets the first aid standard with respect to the environmental background, and recognizing and judging whether a patient human body position feature meets the first aid standard with respect to the human body foreground

S300 Providing CPR instruction and guidance on CPR actions during the CPR implementation

during the CPR implementation, using a human body pose recognition model to position a key point of an implementer and obtain the feature for detecting and positioning the key point of implementer's human body posture, establishing a human body skin reflection model, feeding CPR feedback of a patient back in real time on the feature of patient hemodynamic parameter, and in combination with the feature of detecting and positioning a key point of implementer's human body posture and the feature of patient hemodynamic parameter, using the trained CPR mapping model to output CPR action standard feedback so as to perform instruction on CPR actions and to guide implementation of a CPR process

Fig.1

Fig.2

First aid platform
server

Intelligent AED
apparatus

AI recognition
module

Wireless
communicat
ion

First aid medical
terminal

Warning
apparatus

Fig.3

Awakening
detection module

Communication
module

Voice broadcasting
module

Video collection
module

Audio collection
module

Master control
module

Video display
module

Power module

Early warning
module

AI recognition
module

Storage module

Fig.4

Start

Enabling an intelligent AED apparatus
Starting a remote AI first aid function

Connecting to a remote doctor

Pasting an electrode plate

A whole process AI voice guide the start with

The remote doctor participates in the first aid with a video in a whole process

The AI detects whether the electrode plate is correctly pasted and prompts an error correction

An AED judges whether defibrillation is needed

Starting AED defibrillation

Starting CPR

When the AED judges that CPR needs to be performed on the patient at this moment, the AI intelligent first aid function is started to instruct the user to operate by voice in the whole process, and the whole process is assisted by a video recognition technology, which corrects the whole first aid process.
For example, a rescuer is prompted that "Your CPR press depth does not conform to a standard, and please operate correctly. "

After the AI first aid function is started, AI recognizes whether an environment in which a patient is located conforms to a first aid standard, e.g., the environment is noisy and wet, then the voice prompts "The current environment is not conducive to first aid, please move to a dry and quiet environment for first aid"
The AI then recognizes whether the electrode plate is pasted in a correct position, and if the position is wrong, the voice corrects "No valid data is detected, please replace the electrode plate"

Whether error correction data processing during artificial intelligence-assisted first aid needs to be calculated locally at an edge or uploaded to a cloud server for a calculation is judged by an AI algorithm to ensure real-time feedback

CPR video data is uploaded to the server in real time for a calculation

Algorithm route

The AI detects and prompts a user to place a webcam in a correct position

An algorithm model processes data in real time

AI detects whether the CPR action is standard in real time and corrects an error with a voice prompt

AI detects the CPR action in real time to display accurate data on a screen

Real-time data assists the doctor in decision making

A combination of a human body pose recognition model and a skin reflection model will automatically recognize CPR, and detect and feed back a CPR action in real time

A system compares CPR assessment standard output results

Continue CPR in conjunction with the AI error correction prompt

End

A defibrillation data and CPR data report is sent back to a terminal

Fig.5

EP 4 553 848 A1

Fig.6

Fig. 7

Fig. 8

## INTERNATIONAL SEARCH REPORT

| International application No. |
| --- |
| **PCT/CN2024/079795** |

| A. | CLASSIFICATION OF SUBJECT MATTER |
| --- | --- |
| | G16H50/20(2018.01)i |

According to International Patent Classification (IPC) or to both national classification and IPC

| B. | FIELDS SEARCHED |
| --- | --- |

Minimum documentation searched (classification system followed by classification symbols)

IPC:G16H

Documentation searched other than minimum documentation to the extent that such documents are included in the fields searched

Electronic data base consulted during the international search (name of data base and, where practicable, search terms used)

CNABS, CNTXT, DWPI, VEN, ENTXTC: 动作, 规范, 急救, 皮肤, 图像, 心肺复苏, 血流, 指导, 姿态, AED, CPR, first-aid , feedback, action, standard, identify, guide

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116994746 A (SUZHOU YUYUE MEDICAL TECHNOLOGY CO., LTD. et al.) 03 November 2023 (2023-11-03) claims 1-19 and figure 1 | 1-19 |
| A | CN 112749684 A (XUANWEI (BEIJING) BIOTECHNOLOGY CO., LTD.) 04 May 2021 (2021-05-04) entire document | 1-19 |
| A | CN 115641646 A (XUANWU HOSPITAL, CAPITAL MEDICAL UNIVERSITY) 24 January 2023 (2023-01-24) entire document | 1-19 |
| A | US 2015105637 A1 (PEKING UNION MEDICAL COLLEGE HOSPITAL, CHINESE ACADEMY OF MEDICAL SCIENCES et al) 16 April 2015 (2015-04-16) entire document | 1-19 |
| A | US 2009306525 A1 (KONINKLIJKE PHILIPS ELECTRONICS N.V.) 10 December 2009 (2009-12-10) entire document | 1-19 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **18 April 2024** | **19 April 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2024/079795** |

| Patent document cited in search report | | | Publication date (day/month/year) | Patent family member(s) | | | Publication date (day/month/year) |
| --- | --- | --- | --- | --- | --- | --- | --- |
| CN | 116994746 | A | 03 November 2023 | CN | 116994746 | B | 30 January 2024 |
| CN | 112749684 | A | 04 May 2021 | None | | | |
| CN | 115641646 | A | 24 January 2023 | CN | 115641646 | B | 09 May 2023 |
| US | 2015105637 | A1 | 16 April 2015 | EP | 2859913 | A1 | 15 April 2015 |
| | | | | EP | 2859913 | B1 | 19 August 2020 |
| | | | | US | 2018207058 | A1 | 26 July 2018 |
| | | | | US | 10463566 | B2 | 05 November 2019 |
| | | | | US | 9949892 | B2 | 24 April 2018 |
| | | | | US | 2020009011 | A1 | 09 January 2020 |
| | | | | US | 11471375 | B2 | 18 October 2022 |
| US | 2009306525 | A1 | 10 December 2009 | JP | 2009501041 | A | 15 January 2009 |
| | | | | EP | 1906818 | A2 | 09 April 2008 |
| | | | | WO | 2007010422 | A2 | 25 January 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311256171